# EUROPEAN PATENT APPLICATION

(11) **EP 3 090 745 A1**
(43) Date of publication of application: **09.11.2016**
(21) Application number: 16168477.4
(22) Date of filing: 24.03.2008
(51) Int. Cl.: A61K 31/48, A61P 9/00, A61P 9/04

(54) **METHODS OF TREATING VASCULAR DISEASES USING BROMOCRIPTINE**

(30) Priority: 30.03.2007 US 921113 P; 08.06.2007 US 933753 P; 24.07.2007 US 961747 P; 20.03.2008 US 77552
(62) Divisional of application: 08742225.9
(71) Applicant: VeroScience LLC, Tiverton RI 02878 (US)
(72) Inventor: CINCOTTA, Anthony H, Tiverton, RI Rhode Island 02787 (US)
(74) Representative: Elsy, David

(57) **Abstract**

The present invention is directed to a method of simultaneously treating hypertension, hypertriglyceridemia, a pro-inflammatory state, a pro-coagulative state, and insulin resistance (with or without treating obesity or endothelial dysfunction), associated with or independent from Metabolic Syndrome, as well as vascular disease such as cardiovascular, cerebrovascular, or peripheral vascular disease comprising the step of administering to a patient suffering from such disorders a therapeutically effective amount of a central acting dopamine agonist. In one embodiment, the central acting dopamine agonist is bromocriptine, optionally combined with a pharmaceutically acceptable carrier.

## Description

### BACKGROUND OF THE INVENTION

### 1. Field of the Invention:

This invention relates to methods of treating metabolic disorders, and more particularly, to methods of treating Metabolic Syndrome, its composite individual disordersor manifestations of biochemical abnormalities associated with cardiometabolic risk or metabolic syndrome such as vascular inflammation and endothelial dysfunction that predispose to cardiovascular disease, peripheral vascular disease, or cerebrovascular disease as well as treating these vascular diseases by administering a central acting dopamine agonist such as bromocriptine.

### 2. Description of the Related Art:

Metabolism is a complex orchestration of biochemical processes among cells and tissues of the body all working in concert to ensure the survival of the organism as a whole. The central nervous system plays a major role in integrating these metabolic activities to maintain normal biological homeostasis within the body. Environmental and genetic perturbations to this central nervous system control of metabolism can manifest as a range of metabolic disorders. Additionally, since metabolic processes have profound effects on the entire body, diseases and disorders affecting metabolism generally affect other areas of the body as well. For example, individuals suffering from Type 2 diabetes often experience problems with several body organs and systems. Typically, plasma glucose levels are elevated in Type 2 diabetes as a result of the body's resistance to the glucose-lowering effects of a hormone called insulin as well as to decreased ability to secrete appropriate amount so f insulin after a meal. Type 2 diabetes is associated with damage to various organs such as the eyes, nerves, and kidneys. The disease is also associated with substantially increased risk for cardiovascular disease (CVD), the leading cause of death in Type 2 diabetics. The prevalence of Type 2 diabetes is reaching epidemic proportions in the United States and around the world.

According to the guidelines of the American Diabetes Association, to be diagnosed with Type 2 diabetes, an individual must have a fasting plasma glucose level greater than or equal to 126 mg/dl or a 2-hour oral glucose tolerance test (OGTT) plasma glucose value of greater than or equal to 200 mg/dl (Diabetes Care, 26:S5-S20, 2003). A related condition called pre-diabetes is defined as having a fasting glucose level of greater than 100 mg/dl but less than 126 mg/dl or a 2-hour OGTT plasma glucose level of greater than 140 mg/dl but less than 200 mg/dl. Mounting evidence suggests that the pre-diabetes condition may be a risk factor for developing cardiovascular disease (Diabetes Care 26:2910-2914, 2003).

Metabolic Syndrome (MS), also referred to as Syndrome X, is another metabolic disorder that affects other pathways and systems in the body. Originally, Metabolic Syndrome was defined as a cluster of metabolic disorders (including obesity, insulin resistance, hypertension, and dyslipidemia primarily hypertriglyceridemia), that synergize to potentiate cardiovascular disease. More recently (2001), the U.S. National Cholesterol Education Program (NCEP) has classified Metabolic Syndrome as meeting any three out of the following five criteria: fasting glucose level of at least 110 mg/dl, plasma triglyceride level of at least 150 mg/dl (hypertriglycerdemia), HDL cholesterol below 40 mg/dl in men or below 50 mg/dl in women, blood pressure at least 130/85 mm Hg (hypertension), and central obesity, with central obesity being defined as abdominal waist circumference greater than 40 inches for men and greater than 35 inches for women. Presently, there are three other internationally recognized definitions for Metabolic Syndrome as follows: 1) World Health Organization 2) American Heart Association/National Heart, Lung and blood Institute (AHA/NHLBI) and 3) International Diabetes Federation (IDF). The definitions of Metabolic Syndrome by the WHO, AHA/NHLBI and IDF are very similar to the definition of the NECP and all use the same metabolic parameters to define the syndrome, but the WHO also includes assessment of insulin fasting insulin levels (Moebus S et al, Cardiovascualr Diabetology, 6: 1-10, 2007; Athyros VG et al, Int. J. Cardiology, 117: 204-210, 2007). Yet subtle differences in the thresholds for these metabolic parameters required to be classified as having the syndrome among these different definitions can result in different classification of a particular subject as having or not having the syndrome according to these different definitions. Also, the prevalence of cardiovascular disease (CVD) with MS varies by the definition used. (Moebus S et al, Cardiovascualr Diabetology, 6: 1-10, 2007; Athyros VG et al, Int. J. Cardiology, 117: 204-210, 2007) . Notably, none of these widely utilized definitions of MS employs vascular pro-inflammatory state, pro-coagulative state, pro-oxidant state, or endothelial dysfunction to define the syndrome. However, these non-metabolic biochemical derangements are often associated with MS. A more recent term for MS plus blood vessel pathophysiology (described just above) has been termed cardiometabolic risk. The American Diabetes Association estimates that 1 in every 5 overweight people suffer from Metabolic Syndrome.

While these disorders and diseases are related, it is clear that they have individual and distinct pathologies. For that reason, drugs used to treat one disorder (namely type 2 diabetes) may not be effective against another disorder (namely metabolic syndrome). For instance, drugs that are effective in treating Type 2 diabetes or pre-diabetes have little to no effect on effectively and safely Metabolic Syndrome. Additionally, certain drugs used to treat Type 2 diabetes or pre-diabetes may increase blood pressure (hypertension) or cause weight gain in the individuals taking the medication. For example, thiazolidinediones used in the treatment of Type 2 diabetes cause weight gain and has marginal effects on hypertension. Another anti-diabetic agent, metformin, also has marginal effects on hypertension and hypertriglyceridemia. Insulin, which is a hormone used to treat Type 2 diabetes can potentiate hypertension and weight gain. Moreover, anti-hypertensive drugs do not necessarily treat dyslipidemia or obesity, and many can worsen insulin sensitivity instead of improving it. It is therefore not a forgone conclusion that since a drug is an effective anti-diabetes agent, that it will be an effective treatment for metabolic and/or non-metabolic pathologies of metabolic syndrome. Since people with metabolic syndrome do not have existing disease but have a biology that portends ensuing disease, the criteria for safety are also much higher when considering a pharmaceutical agent for the treatment of this syndrome.

Since the Metabolic Syndrome is diagnosed as having several criteria (as described above) yet also encompasses vascular abnormalities such as endothelial dysfunction, vascular pro-inflammatory condition, and vascular pro-coagulative condition, the treatment of Metabolic Syndrome according to the present invention further includes
a. Treatment of endothelial dysfunction associated with cardiovascular disease;
b. Treatment of hypertension, vascular pro-inflammatory state, and pro-coagulative state simultaneously. Examples of pro-inflammatory state blood markers include but are not limited to: C-reactive protein, serum amyloid A protein, interleukin-6, interleukin-1, Tumor Necrosis Factor-alpha, homocysteine, and white blood cell count. Examples of pro-coagulative state blood markers include but are not limited to: endothelin-1, hematocrit viscosity, red cell aggregation, plasminogen activator inhibitor-1, fibrinogen, van Willebrand factor, Factor VII, Factor VIII, and Factor IX;
c. Treatment of at least two of hypertension, vascular pro-inflammatory state, or pro-coagulative state simultaneously; and
d. Treatment of at least one of hypertension, vascular pro-inflammatory state, or pro-coagulative state.

The endothelium can modify circulating factors as well as synthesize and release factors that influence cardiovascular health and disease. Endothelium dysfunction is characterized by alterations in endothelium modulation of the vasculature that favor or potentiate vasoconstriction, a pro-coagulant state, and/or a pro-inflammatory state as well as other biochemical process that all contribute to the initiation and progression of atherosclerosis (Am. J. Cardiol. 91(12A): 3H-11H, 2003; Am. J, Cardiol. 115 Suppl 8A:99S-106S, 2003) or arteriosclerosis (Nigam A et al"Am. J. Cardiol. 92: 395-399, 2003; Cohn JN et al, Hypertension 46:217-220, 2005; Gilani M et al, J. Am. Soc. Hypertens 2007) depending upon the biochemistry involved.

A variety of treatments are available for diseases associated with obesity, including Type 2 Diabetes. For example, U.S. Patent No. 6,506,799 discloses methods of treating cardiovascular diseases, dyslipidemia, dyslipoproteinemia, and hypertension comprising administering a composition comprising an ether compound. U.S. Patent No. 6,441,036 discloses fatty acid analogous which can be used for the treatment and/or prevention of obesity, fatty liver and hypertension.

U.S. Patent No. 6,410,339 discloses use of cortisol agonist for preparing a system for diagnosis of the Metabolic Syndrome and related conditions as belly fatness, insulin resistance including increased risk of developing senile diabetes, i.e., diabetes type II, high blood fats and high blood pressure, in which system the dose of cortisol agonist is in an interval where a difference is obtained in the inhibitory effect of the autoproduction of cortisol in individuals suffering from the Metabolic Syndrome, compared to normal values.

U.S. Patent No. 6,376,464 discloses peptides and peptide analogues that mimic the structural and pharmacological properties of human ApoA-I. The peptides and peptide analogues are useful to treat a variety of disorders associated with dyslipidemia.

U.S. Patent No. 6,322,976 discloses, among other things, methods of diagnosing a disease associated with a defect in insulin action, glucose metabolism, fatty acid metabolism, and/or catecholamine action by detecting a mutation in the CD36 gene.

U.S. Patent No. 6,197,765 discloses a treatment for Metabolic Syndrome (syndrome-X), and resulting complications, by administration of diazoxide.

U.S. Patent No. 6,166,017 discloses a method for the medical treatment of diabetes mellitus type II and for counteracting the risk factors forming part of the Metabolic Syndrome by administration of ketoconazole.

U.S. Patent No. 6,040,292 discloses methods for the treatment of diabetes mellitus, including type I, type II, and insulin resistant diabetes (both type I and type II). The methods of the invention employ administration of rhIGF-I/IGFBP-3 complex to a subject suffering from the symptoms of diabetes mellitus. Administration of rhIGF-I/IGFBP-3 to a subject suffering from the symptoms of diabetes mellitus results in amelioration or stabilization of the symptoms of diabetes.

U.S. Patent No. 5,877,183 discloses methods for the regulation and modification of lipid and glucose metabolism, but not Metabolic Syndrome, by administering to a subject a dopamine D1 agonist, optionally in combination with a dopamine D2 agonist, an alpha-1 adrenergic antagonist, an alpha-2 adrenergic agonist, or a serotonergic inhibitor, or optionally in combination with a dopamine D2 agonist coadministered with at least one of alpha-1 adrenergic antagonist, an alpha-2 adrenergic agonist, or a serotonergic inhibitor, and further administering the subject a serotonin 5HT_{1b} agonist. It is well known that dopamine agonists function to both activate and deactivate dopamine receptors and thereby reduce dopaminergic neuronal activity.

U.S. Patent No. 5,741,503 discloses methods for regulating or ameliorating lipid metabolism which comprise administration or timed administration of inhibitors of dopamine beta hydroxylase (DBH). However, the focus of this technology is reduction in noradrenergic neuronal activity level only and does not increase dopaminergic neuronal activity inasmuch as DBH is not present in dopaminergic neurons that are anatomically distinct from noradrenergic neurons where DBH resides.

In addition, several U.S. Patents disclose use of dopamine agonists such as bromocriptine for use in treating pathologies relating to Type II diabetes. See, for example, US Patent Nos. 6,004,972; 5,866,584; 5,756,513; and 5,468,755. Also, bromocriptine has been employed to treat type 2 diabetes or insulin resistance (Pijl H, et al Diabetes Care, 23:1154, 2000; Meier AH et al, Diabetes Reviews, 4: 464, 1996).

A significant complicating issue in the treatment of metabolic disorders is that the individual pathologies of Metabolic Syndrome differ in their nature and magnitude whether presented alone or as part of the syndrome because the pathologies of the syndrome tend to synergize to produce increased risk of morbidity and mortality (Reviewed in GM Reaven, Diabetes, Obesity, and Metabolism, 4: (Suppl. 1) S13-S-18, 2002). In other words, a Metabolic Syndrome subject carries a different increased risk of cardiovascular disease as a result of his/her hypertension than does a hypertensive subject without Metabolic Syndrome. Currently, the U. S. Food and Drug Administration has not approved the use of any drug for the treatment of Metabolic Syndrome. The current definition of Metabolic Syndrome by the NCEP and the other definitions as described above relates to metabolic derangements and does not include aspects of non-metabolic biochemical pathology associated with the Syndrome such as pro-coagulative state, pro-inflammatory state, pro-oxidant state, or endothelial dysfunction. Yet these non-metabolic biochemical derangements contribute significantly to cardiovascular disease by mechanisms that do not necessarily involve lipid deposition and its attendant consequences of plaque formation in the intimal and inner media vessel walls (i.e., atherosclerosis). Rather, these non-metabolic biochemical abnormalities can potentiate a process that leads to a different type of vascular damage termed arterioscleosis (defined as thickening and stiffening of the vessel wall) that can have devastating consequences on vascular health and potentiate vascular disease such as large vessel damage, myocardial infarction, stroke, and peripheral vascular disease (Safar ME Frohlich ED (eds) Atherosclerosis, Large Arteries and Cardiovascular Risk. McEniery CM et al, Adv. Cardiol. Basel, Karger, vol. 44, pp. 160-172; Laurent S et al, Eur. Heart J., 27: 2588-2605, 2006). These non-metabolic biochemical pathologies predispose the individual to increased stiffening of the vessel wall by changing the biochemical structure and architecture within the cellular layers of the wall (i.e., extracellular matrix components such as collagen and elastin, etc.) and by changing the contractile state of the smooth muscle cells therein (Safar ME Frohlich ED (eds) Atherosclerosis, Large Arteries and Cardiovascular Risk. McEniery CM et al, Adv. Cardiol. Basel, Karger, vol. 44, pp. 160-172). Such changes can effectuate vascular damage often in a much shorter time frame than those metabolic derangements of Metabolic Syndrome predisposing to atherosclerosis. Moreover, these non-metabolic derangements can be additive to those metabolic disturbances defining the Metabolic Syndrome to exacerbate vascular disease. And, arteriosclerosis can predispose one to atherosclerosis. Since arteriosclerosis often precedes and potentiates atherosclerosis, the ability to successfully treat arteriosclerosis or biochemical events leading to arteriosclerosis, on e may be able to intervene medically at an earlier time point in the chronology of CVD and produce better clinical outcomes for the patient in the long term.

The mechanisms involving non-metabolic biochemical derangements of a vascular pro-inflammatory state, pro-oxidant state, pro-coagulative state, and endotheial dysfunction to precipitate arterioscleosis and CVD are exceedingly complex and reviewed in much detail in Nigam A et al"Am. J. Cardiol. 92: 395-399, 2003; Cohn JN et al, Hypertension 46:217-220, 2005; and Gilani M et al, J. Am. Soc. Hypertens 2007.

Previous studies have described the utility of the dopamine agonist, bromocriptine to treat individual pathologies of insulin resistance, hypertension, hypertriglyceridemia individually but not as a composite and also to treat lipid plaques of atherosclerosis (Meier AH et al, Diabetes Reviews, 4: 464, 1996; U.S. patent #s 5,006,526 and 5,565,454). However, to our knowledge no literature are available describing the utility of bromocriptine or dopamine agonists to simultaneously treat metabolic derangements of MS *and* non-metabolic derangements associated with MS or to simultaneously treat several non-metabolic derangements associated with MS or to treat arteriosclerosis (as opposed to atherosclerosis) or to reduce actual adverse cardiovascular events such as myocardial infarction , stroke, angina or peripheral vascular disease (or increase time to these adverse events). Moreover, although timing of administration to effectuate improvements in metabolic derangements such as type 2 diabetes and insulin resistance has been described (US Patent Nos. 6,004,972; 5,866,584; 5,756,513; and 5,468,755), such import of circadian timing to maximize the benefit of dopamine agonist therapy upon non-metabolic biochemical activities predisposing to arteriosclerosis and CVD that are wholly different from those metabolic influences as previously described in the literature, have not been delineated. In fact, the available literature indicate that dopamine agonist therapy such as bromocriptine is associated with *increased* adverse cardiovascular events such as myocardial infarction, stroke, and cerebrovascular accident (Ruch A et al, Obstet Gynecol 74: 448-451, 1989; Iffy L et al, Med Law 15: 127-134, 1996; Katz M et al, Obstet Gynecol 66: 822-824, 1985; Iffy et al, Am J Ther 5: 111-115, 1998; Ddutt S et al, Aust N Z J Obstet Gynaecol 38: 116-117, 1998). In fact, the effect of dopamine agonists such as bromocriptine to *increase* these adverse cardiovascular events was serious enough for the U.S. Food and Drug Administration to place a warning on the labels for these pharmaceutical dopamine agonists stating that their use has been associated with increases in hypertension, stroke, cerebrovascular accidents, and myocardial infarction (Physicians Desk Reference, Parlodel Package Insert). In stark contradistinction to this described relationship between increased dopamine agonist exposure and increased vascular disease, the current invention demonstrates that if the dopamine agonist therapy is used at the appropriate dosage and at the appropriate time of day so that its levels are not elevated throughout a greater portion of the day but are confined to a discrete daily interval of the day that approximates the natural daily circadian peak of central nervous system dopaminergic activity in healthy individuals without either vascular disease or increased levels of metabolic or non-metabolic biomarkers of vascular disease and given to a subject in need of treatment for cardiovascular disease, then dopamine agonist therapy actually *decreases* vascular disease and adverse vascular events, not *Increases* them. Such daily timing of dopamine agonist within the present invention to improve arteriosclerosis biomarkers, arteriosclerosis, and CVD events also is at a time of day to reduce exaggerated increases in central noradrenergic tone that potentiate these vascular disorders. And, these beneficial vascular effects of timed dopamine agonist therapy are not the result of influences to markedly reduce hyperglycemia, plasma triglyceride levels, or blood pressure (see examples below).

The vascular endothelium is a dynamic tissue, responding to the humoral milieu it is bathed in to impact vascular architecture, and blood vessel contractile tone. Endothelial dysfunction may be defined as a biochemical state wherein the endothelium potentiates vasoconstriction, inflammation of the vessel wall intima and media layers, and physical restructuring of the extracellular matrix of the vessel wall to potentiate wall thickening and stiffening. Among the humoral factors known to stimulate biochemical endothelial dysfunction, increases in pro-inflammatory factors such as monocyte chemoattractant protein - 1 (MCP-1), tumor necrosis factor- alpha (TNFalpha), interleukin- 6 (IL-6) and C-reactive protein (CRP) all stimulate endothelial changes that facilitate inflammation at the vessel wall that in turn potentiate vessel wall stiffening. Moreover, decreases in plasma adiponectin, an anti-inflammatory factor at the vessel wall, also facilitate endothelial dysfunction and inflammation at the endothelium thereby potentiating vessel wall stiffening (i.e., arterioscleosis). Vascular inflammation is coupled to and facilitates arterial stiffness (Yasmin MC et al, Arterioscler. Thromb. Vasc. Biol. 24: 969-974, 2004; Duprez DA et al, J. Hum. Hypertens. 19: 515-519, 2005; Booth A et al, Arthritis Rheum. 50: 581-588, 2004).

Vascular oxidative stress can also contribute to arterial wall stiffness. Increases in oxidative stress that produce reactive oxygen species (ROS) can scavenge nitric oxide, a potent endothelium stimulus for vasodilatation and normal endothelium function. Reduced vascular nitric oxide (NO) availability can potentiate arterial wall stiffness and a direct correlation between arterial stiffness and endothelial function has been observed in both the coronary and peripheral circulations (Wilkinson IB et al, Circulation 105: 213-217, 2002; Schmitt M et al, Hypertension 46: 227-231, 2005; Ichigi Y et al, J. Am. Coll. Cardiol. 45: 1461-1466, 2005; Ceravolo R et al, J. Am. Coll. Cardiol. 41: 1753-1758, 2003). Endothelial dysfunction and reduced NO availability can derive from too little NO synthase activity or from a consequence of over-active but "uncoupled" NO synthase activity. Paradoxically, vascular NO synthase expression may be increased in states of endothelial dysfunction and vascular disease. In the consequence of increased uncoupled vascular NO synthase activity, the enzyme functions to generate increased ROS and protein tyrosine nitration in the vessel wall while reducing the amount of available NO that collectively potentiate vascular arterioscleosis (Upmacis RK et al, Am. J. Physiol. 293: H2878-2887, 2007; Ginnan R et al, Free Radic. Biol. Med., Jan 22, 2008; Landmesser et al, J. Clin. Invest., 111: 1201-1209, 2003; Munzel T et al, Arterioscler. Thromb. Vasc. Biol., 25: 1551-1557, 2005). Beyond their influence on inflammation, the above described adipokines (increased TNFalpha and MCP-1 and decreased adiponectin) and increased CRP, also may potentiate increases in ROS and protein nitration via perturbations of endothelial function and NO synthase (Rong L et al, Am. J. Physiol. 293: E1703-E1708, 2007; De Keulenger GW et al, Biochem. J. 329: 653-657, 1998). Increases in vessel endothelial NO synthase (eNOS) (Kagota S et al, Life Sciences 78:1187-1196, 2006) and inducible NO synthase (iNOS) are observed in older SHR rats that have increased arterial stiffness (Safar ME, In: Swales JD ed., Textbook of Hypertension, London UK: Blackwell Scientific ; 1994:85-102). In the case of increased "uncoupled" NO synthase activity, the uncoupled NO synthase actually produces increased local amounts of superoxide while reducing its NO production thereby contributing to arteriosclerosis and this occurrence appears to be particularly accentuated in diabetes (Alp NJ et al, J. Clin. Invest. 112: 725-735, 2003) and may contribute significantly to the arteriosclerosis of diabetes and the consequent increase in cardiovascular events (MI, stroke, and peripheral vascular damage) of diabetes versus non-diabetes subjects. A key hallmark of eNOS uncoupling is an increase in eNOS level or activity with a concurrent decrease in soluble guanyl cyclase level or activity in the endothelium as this enzyme is activated by NO to induce NO beneficial effects on the vasculature.

A pro-coagulative state also can predispose one to increased cardiovascular events. Respecting acute coronary syndrome, acute myocardial infarction, and thrombotic stroke, a critical player in their genesis is a pro-coagulative state, a condition potentiating an increase in the balance between blood clot formation and blood clot dissolution favoring blood clot formation. A pro-coagulative state involves many biochemical factors within the physiology of the body and increases in factors that potentiate blood clot formation and/or inhibit blood clot dissolution can function not only to precipitate an acute CVD event, but also can function to facilitate mechanisms involved in arteriosclerosis as well. Endothelin-1, is an example of such a factor. Endothelin-1 is an endothelium derived factor that is very pro-coagulative and that also functions as a potent vasoconstrictor that can potentiate endothelial dysfunction (Halim A et al, Thromb REs 72: 203-209, 1993; Iwamoto T et al, Nephron 73: 273-279, 1996) and thereby lead to arterial stiffness. Various factors in clot formation such as reactive platelets, plasminogen activator inhibitor-1, and fibrinogen, synergize to alter the endothelium and vessel wall in chronic hyper-coagulative states that can lead to vessel wall restructuring, chronic vasoconstriction and arteriosclerosis.

Endothelial dysfunction as described above may be defined as a biochemical state wherein the endothelium potentiates vasoconstriction, inflammation of the blood vessel wall intima and media layers, and physical restructuring of the extracellular matrix of the blood vessel wall to potentiate wall thickening and stiffening. As such, endothelial dysfunction as defined herein is a potent contributor to arterioscleosis and CVD (Nigam A et al"Am. J. Cardiol. 92: 395-399, 2003; Cohn JN et al, Hypertension 46:217-220, 2005; Gilani M et al, J. Am. Soc. Hypertens 2007). This is an important distinction because those biochemical derangements that affect arteriosclerosis versus atherosclerosis will have distinct beneficial impacts on CVD outcomes. Arteriosclerosis is often a very early sign of later CVD events long before any atherosclerosis is detectable (Nigam A et al"Am. J. Cardiol. 92: 395-399, 2003; Cohn JN et al, Hypertension 46:217-220, 2005; Gilani M et al, J. Am. Soc. Hypertens 2007). Therefore it may be possible to prophylacticly treat one with signs of arteriosclerosis such as endothelial dysfunction, a pro-inflammatory state, a pro-coagulative state, or a pro-oxidant state, which are all easily assessable clinically, in an effort to best prevent the onset of arteriosclerosis or CVD by attacking the problem at the time of its earliest warning signs. There are several simple tests to measure endothelial dysfunction, a vascular pro-inflammatory state, a pro-coagulative state, and a pro-oxidant state. Also, there are several available test to assess presence and degree of arteriosclerosis. It is also true that certain other biochemical derangements within the endothelium may also predispose one to atherosclerosis, however, as it relates to this invention, and as it is defined herein, endothelial dysfunction is a factor that potentiates arteriosclerosis. It can be appreciated that endothelial dysfunction will be characterized by biochemical derangements of the endothelium including but not limited to increased "uncoupled" inducible NO synthase, "uncoupled" endothelial NO synthase, increased ROS, increased production of and/or exposure to vasoconstrictive factors such as Endothelin-1, and increased presence and activity of pro-inflammatory and pro-coagulative factors.

The metabolic derangements that define the metabolic syndrome as described above differ in their impact on CVD from the non-metabolic derangements described above. Statins, drugs that reduce total and low-density lipoprotein (LDL) cholesterol synthesis by inhibiting HMG-CoA reductase activity and fibrates that reduce plasma triglyceride levels have been shown to reduce blood vessel plaques and CVD events (Colhoun H et al, Lancet 364; 685-696, 2004). Also, anti-hypertensive medications have been shown to reduce CVD events (Sever P et al, Lancet 361: 1149-1158, 2003). However, cardiovascular disease still remains the leading cause of morbidity in the world today and in subjects with type 2 diabetes cardiovascular disease is the leading cause of death. Moreover, in this diabetes patient population, CVD events have been increasing in recent years despite the availability of statins, fibrates and anti-hypertensive medications (Roglic G et al, Diabetes Care, 28: 2130 -2135, 2005). Clearly these medications are not completely effective and new methods of preventing CVD and treating CVD are needed. Particularly, an effective treatment for the metabolic pathologies of metabolic syndrome and non-metabolic pathologies associated with metabolic syndrome to effectuate a prevention of, improvement in, reduction of the progression of, or regression of arteriosclerosis and CVD is needed. Methods that reduce arteriosclerosis as well as atherosclerosis and biological potentiators of both these vascular disorders are also needed. Moreover, these methods are particularly needed in subjects with type 2 diabetes. The present invention is believed to be an answer to these needs.

### BRIEF SUMMARY OF THE INVENTION

Disclosed herein are examples of the invention, including:-
1. A method of treating at least one non-metabolic derangement in a patient, comprising the step of administering to a patient suffering from said non-metabolic derangement a therapeutically effective amount of a central acting dopamine agonist, said central acting dopamine agonist effective to treat said at least one non-metabolic derangement in said patient.
2. The method of clause 1, wherein said non-metabolic derangement is selected from the group consisting of a vascular pro-inflammatory state, pro-coagulative state, pro-oxidant state, or endothelial dysfunction.
3. The method of clause 1, wherein said patient also suffers from Metabolic Syndrome or Type 2 diabetes, and said method also treats Metabolic Syndrome and/or Type 2 diabetes.
4. The method of clause 1, wherein the central acting dopamine agonist is selected from dopamine D2 receptor agonists and/or dopamine D1 receptor agonists.
5. The method of clause 4, wherein said dopamine agonist is an ergot-related compound.
6. The method of clause 5, wherein wherein said ergot-related compound has low or no serotonin receptor 2B agonist activity.
7. The method of clause 5, wherein said ergot-related compound is selected from the group consisting of bromocriptine, lisuride, dihydroergotoxine, dihydro-alpha-ergocryptine, terguride, and combinations thereof.
8. The method of clause 4, wherein the central acting dopamine agonist is selected from the group consisting of quinpirole, quinerolane, talipexole, ropinirole, apomorphine, fenoldopam, benzazepine analogs, and combinations thereof.
9. The method of clause 1, wherein said dopamine agonist is administered at a predetermined time of day.
10. The method of clause 9, wherein said dopamine agonist is administered so as to effectuate peak plasma levels of the dopamine agonist between 0400 and 1200 hours of the day.
11. The method of clause 1, wherein said dopamine agonist bioavailability in the blood is reduced to within about 50% of the plasma peak value from about 2 to 6 hours after the end of the daily peak or plateau plasma level of dopamine agonist.
12. The method of clause 1, wherein said dopamine agonist is administered in conjunction with at least one of other anti-diabetes agent, anti-obesity agent, antihypertensive agent, anti-inflammatory agent, or cholesterol lowering agent.
13. The method of clause 1, wherein said method further comprises treating obesity.
14. The method of clause 1, wherein said therapeutically effective amount of a central acting dopamine agonist ranges from 0.001 mg per kg body weight to 2.0 mg per kg body weight.
15. A method of treating at least one metabolic derangement and at least one non-metabolic derangement in a patient, comprising the step of administering to a patient suffering from said metabolic derangement and said non-metabolic derangement a therapeutically effective amount of a central acting dopamine agonist, said central acting dopamine agonist effective to treat said at least one metabolic derangement and said at least one non-metabolic derangement in said patient.
16. The method of clause 15, wherein said non-metabolic derangement is selected from the group consisting of a vascular pro-inflammatory state, pro-coagulative state, pro-oxidant state, or endothelial dysfunction.
17. The method of clause 15, wherein said metabolic derangement is selected from the group consisting of insulin resistance, hypertriglyceridemia, and hypertension.
18. The method of clause 15, wherein said patient also suffers from Metabolic Syndrome or Type 2 diabetes, and said method also treats Metabolic Syndrome and/or Type 2 diabetes.
19. The method of clause 15, wherein the dopamine agonist is selected from dopamine D2 receptor agonists and/or dopamine D1 receptor agonists.
20. The method of clause 15, wherein said dopamine agonist is an ergot-related compound.
21. The method of clause 20, wherein wherein said ergot-related compound has low or no serotonin receptor 2B agonist activity.
22. The method of clause 20, wherein said ergot-related compound is selected from the group consisting of bromocriptine, lisuride, dihydroergotoxine, dihydro-alpha-ergocryptine, terguride, and combinations thereof.
23. The method of clause 15, wherein the central acting dopamine agonist is selected from the group consisting of quinpirole, quinerolane, talipexole, ropinirole, apomorphine, fenoldopam, benzazepine analogs, and combinations thereof.
24. The method of clause 15, wherein said dopamine agonist is administered at a predetermined time of day.
25. The method of clause 24, wherein said dopamine agonist is administered so as to effectuate peak plasma levels of the dopamine agonist between 0400 and 1200 hours of the day.
26. The method of clause 15, wherein said dopamine agonist bioavailability in the blood is reduced to within about 50% of the plasma peak value from about 2 to 6 hours after the end of the daily peak or plateau plasma level of dopamine agonist
27. The method of clause 15, wherein said dopamine agonist is administered in conjunction with at least one of other anti-diabetes agent, anti-obesity agent, antihypertensive agent, anti-inflammatory agent, or cholesterol lowering agent.
28. The method of clause 15, wherein said method further comprises treating obesity.
29. The method of clause 15, wherein said therapeutically effective amount of a central acting dopamine agonist ranges from 0.001 mg per kg body weight to 2.0 mg per kg body weight.
30. A method of treating at least one vascular disease in a patient, comprising the step of administering to a patient suffering from or exhibiting biomarkers of said at least one vascular disease a therapeutically effective amount of a central acting dopamine agonist, said central acting dopamine agonist effective to treat said at least one vascular disease in said patient.
31. The method of clause 30, wherein said vascular disease is selected from the group consisting of cardiovascular disease, microvascular disease, macrovascular disease, peripheral vascular disease, and cerebrovascular disease.
32. The method of clause 31, wherein said cardiovascular disease is selected from the group consisting of arteriosclerosis, myocardial infarction, stroke, angina, and congestive heart failure.
33. The method of clause 30, wherein said patient also suffers from one or more of Metabolic Syndrome, endothelial dysfunction, or Type 2 diabetes, and said method also treats Metabolic Syndrome and/or Type 2 diabetes.
34. The method of clause 30, wherein the central acting dopamine agonist is selected from dopamine D2 receptor agonists and/or dopamine D1 receptor agonists.
35. The method of clause 30, wherein said dopamine agonist is an ergot-related compound.
36. The method of clause 35, wherein wherein said ergot-related compound has low or no serotonin receptor 2B agonist activity.
37. The method of clause 35, wherein said ergot-related compound is selected from the group consisting of bromocriptine, lisuride, dihydroergotoxine, dihydro-alpha-ergocryptine, terguride, and combinations thereof.
38. The method of clause 30, wherein the central acting dopamine agonist is selected from the group consisting of quinpirole, quinerolane, talipexole, ropinirole, apomorphine, fenoldopam, benzazepine analogs, and combinations thereof.
39. The method of clause 30, wherein said dopamine agonist is administered at a predetermined time of day.
40. The method of clause 39, wherein said dopamine agonist is administered so as to effectuate peak plasma levels of the dopamine agonist between 0400 and 1200 hours of the day.
41. The method of clause 30, wherein said dopamine agonist bioavailability in the blood is reduced to within about 50% of the plasma peak value from about 2 to 6 hours after the end of the daily peak or plateau plasma level of dopamine agonist.
42. The method of clause 30, wherein said dopamine agonist is administered in conjunction with at least one of other anti-diabetes agent, anti-obesity agent, antihypertensive agent, anti-inflammatory agent, or cholesterol lowering agent.
43. The method of clause 30, wherein said method further comprises treating obesity.
44. The method of clause 30, wherein said therapeutically effective amount of a central acting dopamine agonist ranges from 0.001 mg per kg body weight to 2.0 mg per kg body weight.
45. A method of treating Metabolic Syndrome in a patient, comprising the step of administering to a patient suffering from Metabolic Syndrome a therapeutically effective amount of a central acting dopamine agonist, said central acting dopamine agonist effective to treat said Metabolic Syndrome in said patient.
46. The method of clause 45, wherein said patient also suffers from Type 2 diabetes, and said method also treats Type 2 diabetes
47. The method of clause 45, wherein the central acting dopamine agonist is selected from dopamine D2 receptor agonists and/or dopamine D1 receptor agonists.
48. The method of clause 45, wherein said dopamine agonist is an ergot-related compound.
49. The method of clause 48, wherein wherein said ergot-related compound has low or no serotonin receptor 2B agonist activity.
50. The method of clause 48, wherein said ergot-related compound is selected from the group consisting of bromocriptine, lisuride, dihydroergotoxine, dihydro-alpha-ergocryptine, terguride, and combinations thereof.
51. The method of clause 45, wherein the central acting dopamine agonist is selected from the group consisting of quinpirole, quinerolane, talipexole, ropinirole, apomorphine, fenoldopam, benzazepine analogs, and combinations thereof.
52. The method of clause 45, wherein said dopamine agonist is administered at a predetermined time of day.
53. The method of clause 52, wherein said dopamine agonist is administered so as to effectuate peak plasma levels of the dopamine agonist between 0400 and 1200 hours of the day.
54. The method of clause 45, wherein said dopamine agonist bioavailability in the blood is reduced to within about 50% of the plasma peak value from about 2 to 6 hours after the end of the daily peak or plateau plasma level of dopamine agonist.
55. The method of clause 45, wherein said dopamine agonist is administered in conjunction with at least one of other anti-diabetes agent, anti-obesity agent, antihypertensive agent, anti-inflammatory agent, or cholesterol lowering agent.
56. The method of clause 45, wherein said method further comprises treating obesity.
57. The method of clause 45, wherein said therapeutically effective amount of a central acting dopamine agonist ranges from 0.001 mg per kg body weight to 2.0 mg per kg body weight.
58. A method of simultaneously treating Type-2 Diabetes or obesity and one or more of hypertension, a pro-inflammatory state, a pro-coagulative state, a pro-oxidant state, or endothelial dysfunction, said method comprising the step of administering to a patient a therapeutically effective amount of a central acting dopamine agonist to simultaneously treat Type-2 Diabetes and one or more of hypertension, hypertriglyceridemia, a pro-inflammatory state, and insulin resistance.
59. A method of simultaneously treating Type-2 Diabetes, insulin resistance, hypertriglyceridemia and one or more of hypertension, a pro-inflammatory state, a pro-coagulative state, a pro-oxidant state, or endothelial dysfunction, said method comprising the step of administering to a patient a therapeutically effective amount of a central acting dopamine agonist to simultaneously treat Type-2 Diabetes and one or more of hypertension, hypertriglyceridemia, a pro-inflammatory state, and insulin resistance.

In one aspect, the present invention is directed to a method of simultaneously treating hypertension, hypertriglyceridemia, a pro-inflammatory state, and insulin resistance associated with Metabolic Syndrome, the method comprising the step of administering to a patient suffering with Metabolic Syndrome a therapeutically effective amount of a central acting dopamine agonist to simultaneously treat hypertension, hypertriglyceridemia, a pro-inflammatory state, and insulin resistance.

In another aspect, the present invention is directed to a method of simultaneously treating hypertension, hypertriglyceridemia, a pro-inflammatory state, and insulin resistance associated with Metabolic Syndrome, the method comprising the step of administering to a patient suffering with Metabolic Syndrome a therapeutically effective amount of a pharmaceutical composition comprising bromocriptine and a pharmaceutically acceptable carrier to simultaneously treat hypertension, hypertriglyceridemia, a pro-inflammatory state, and insulin resistance.

In another aspect, the present invention is directed to a method for simultaneously treating hypertension, hypertriglyceridemia, a pro-inflammatory state, a pro-coagulative state, and insulin resistance associated with the Metabolic Syndrome, the method comprising the step of administering to a patient suffering from Metabolic Syndrome a therapeutically effective amount of a central acting dopamine agonist to simultaneously treat hypertension, hypertriglyceridemia, a pro-inflammatory state, a pro-coagulative state, and insulin resistance.

In another aspect, the present invention is directed to a method for simultaneously treating hypertension, a pro-inflammatory state, a pro-coagulative state, and a pro-oxidant state associated with the Metabolic Syndrome, the method comprising the step of: administering to a patient suffering from Metabolic Syndrome a therapeutically effective amount of a central acting dopamine agonist to simultaneously treat hypertension, a pro-inflammatory state, a pro-coagulative state, a pro-oxidant state, and any combination thereof. A pro-oxidant state is defined as a biochemical milieu of increased reactive oxygen species or reactive nitrogen species at the tissue level.

In another aspect, the present invention is directed to a method for simultaneously treating hypertension, a pro-inflammatory state, and a pro-coagulative state the method comprising the step of : administering to a patient suffering from hypertension, a pro-inflammatory state, and a pro-coagulative state, a therapeutically effective amount of a central acting dopamine agonist to simultaneously treat hypertension, a pro-inflammatory state, a pro-coagulative state, a pro-oxidant state, and any combination thereof.

In another aspect, the present invention is directed to a method for treating at least one of hypertension, a pro-inflammatory state, and a pro-coagulative state, or a pro-oxidant state associated with the Metabolic Syndrome, the method comprising the step of administering to a patient suffering from Metabolic Syndrome or not, a therapeutically effective amount of a central acting dopamine agonist to treat at least one of hypertension, a pro-inflammatory state, a pro-coagulative state, and a pro-oxidant state.

In another aspect, the present invention is directed to a method for treating at least two of hypertension, a pro-inflammatory state, and a pro-coagulative state the method comprising the step of administering to a patient suffering from at least one of hypertension, a pro-inflammatory state, and a pro-coagulative state, a therapeutically effective amount of a central acting dopamine agonist to treat at least one of hypertension, a pro-inflammatory state, and a pro-coagulative state.

In another aspect, the present invention is directed to a method for treating endothelial dysfunction associated with the Metabolic Syndrome, the method comprising the step of administering to a patient suffering from Metabolic Syndrome or not a therapeutically effective amount of a central acting dopamine agonist to treat endothelial dysfunction.

In another aspect, the present invention is directed to a method for treating endothelial dysfunction associated with cardiovascular disease, the method comprising the step of administering to a patient suffering from endothelial dysfunction, a therapeutically effective amount of a central acting dopamine agonist to treat endothelial dysfunction.

In another aspect, the present invention is directed to a method for simultaneously treating hypertension, hypertriglyceridemia, a pro-inflammatory state, a pro-coagulative state, insulin resistance, a pro-oxidant state, and endothelial dysfunction associated with the Metabolic Syndrome or not, the method comprising the step of administering to a patient suffering from Metabolic Syndrome or not a therapeutically effective amount of a central acting dopamine agonist to simultaneously treat hypertension, hypertriglyceridemia, a pro-inflammatory state, a pro-coagulative state, insulin resistance, a pro-oxidant state, and endothelial dysfunction.

In another aspect, the invention is directed to a method for treating at least one of metabolic derangements consisting of insulin resistance or hypertriglyceridemia or hypertension and at least one of non-metabolic derangements consisting of a pro-inflammatory state, a pro-coagulative state, a pro-oxidant state, or endothelial dysfunction the method comprising the step of administering to a patient suffering from Metabolic Syndrome or not a therapeutically effective amount of a central acting dopamine agonist to treat at least one of metabolic derangements consisting of insulin resistance or hypertriglyceridemia or hypertension and at least one of non-metabolic derangements consisting of a pro-inflammatory state, a pro-coagulative state, a pro-oxidant state, or endothelial dysfunction.

In another aspect, the invention is directed to a method for treating at least one of non-metabolic derangements consisting of a vascular pro-inflammatory state, a pro-coagulative state, a pro-oxidant state, or endothelial dysfunction associated with metabolic syndrome or not the method comprising the step of administering to a patient suffering from Metabolic Syndrome or not a therapeutically effective amount of a central acting dopamine agonist to treat at least one of non-metabolic derangements consisting of a pro-inflammatory state, a pro-coagulative state, a pro-oxidant state, or endothelial dysfunction.

In another aspect, the present invention is directed to a method for treating, preventing, delaying, retarding or slowing the progression of arteriosclerosis the method comprising the step of administering to a patient suffering from Metabolic Syndrome or not a therapeutically effective amount of a central acting dopamine agonist to treat or prevent arteriosclerosis.

In another aspect, the present invention is directed to a method for treating, preventing, delaying, retarding or slowing the progression of vascular disease, including cardiovascular disease, myocardial infarction, cerebrovascular disease, stroke, angina, or peripheral vascular disease comprising the step of administering to a patient in need of such treatment a therapeutically effective amount of a central acting dopamine agonist to treat such vascular disease. Surprisingly it was found that the magnitude of the beneficial effect derived from such dopamine agonist therapy upon vascular disease is very large (see example 3 below) and greater than one would predict from available evidence of dopamine agonist effects on hyperglycemia or dyslipidemia or hypertension.

In another aspect, the invention relates to treating aspects of the above delineated pathologies and disorders simultaneously to treating type 2 diabetes.

In another aspect, the present invention is directed to a method of a) simultaneously treating hypertension, hypertriglyceridemia, a pro-inflammatory state, a pro-coagulative state, a pro-oxidant state, and insulin resistance, b) simultaneously treating three or more of hypertension, hypertriglyceridemia, a pro-inflammatory state, a pro-coagulative state, a pro-oxidant state, and insulin resistance, c) treating Metabolic Syndrome, d) simultaneously treating Type-2 Diabetes and Metabolic syndrome, e) simultaneously treating Type-2 Diabetes and one or more of hypertension, hypertriglyceridemia, a pro-inflammatory state, , a pro-coagulative state, a pro-oxidant state, and insulin resistance, f) treating endothelial dysfunction associated with the Metabolic Syndrome or g) treating endothelial dysfunction associated with cardiovascular disease, h) treating at least one of non-metabolic derangements consisting of a vascular pro-inflammatory state, a pro-coagulative state, a pro-oxidant state, or endothelial dysfunction associated with metabolic syndrome or not i) treating at least one of metabolic derangements consisting of insulin resistance or hypertriglyceridemia or hypertension and at least one of non-metabolic derangements consisting of a pro-inflammatory state, a pro-coagulative state, a pro-oxidant state, or endothelial dysfunction associated with metabolic syndrome or not j) treating, preventing, delaying, retarding or slowing the progression of arteriosclerosis k) treating, preventing, delaying, retarding or slowing the progression of vascular disease, including cardiovascular disease, myocardial infarction, cerebrovascular disease, stroke, angina, or peripheral vascular disease, the method comprising the step of administering to a patient a therapeutically effective amount of a central dopamine agonist, for example such as a pharmaceutical composition comprising bromcriptine and a pharmaceutically acceptable carrier, at a first predetermined time of day. And furthermore, the present invention is directed to a method of treating the aforementioned vascular disease related conditions wherein the central dopamine agonist is administered in a manner to effectuate a peak in the plasma level of the dopamine agonist during a discrete daily interval that approximates the time of the daily peak in hypothalamic dopaminergic activity of a healthy mammal of the same species. Moreover, the present invention is directed to a method of treating a human with the aforementioned conditions wherein the central dopamine agonist is administered in a manner to effectuate a peak in the plasma level of the dopamine agonist during a discrete daily interval from about 0400 to 1200 hours. Also, the present invention is directed to a method of treating a human with the aforementioned conditions wherein the central dopamine agonist is administered in a manner to effectuate a peak in the plasma level of the dopamine agonist during a discrete daily interval from about 0400 to 1200 hours and thereafter reducing its plasma level to within about 50% of the plasma peak value from about 2 to 6 hours after the end of the daily peak or plateau plasma level of dopamine agonist.

As defined herein, the term "non-metabolic derangement" refers to biomarkers for vascular diseases, including, but not limited to, pro-inflammatory state, a pro-coagulative state, a pro-oxidant state, or endothelial dysfunction. A biomarker is further defined as a physiological condition or biological entity (molecule(s)) that is diagnostic or predictive of increased risk of a future vascular-related disease state.

As defined herein, the term "treating" includes reducing the rate of progression of, or increasing the time to onset of, a selected disease state, as well as a reduced need for revascularization surgery in a patient in need of such treatment.

These and other aspects will be described in more details in the following detailed description of the invention.

### BRIEF DESCRIPTION OF THE DRAWINGS

The invention will be better understood when the following detailed description is taken in conjunction with the several drawings in which:
Figure 1 is a graph showing plasma insulin concentration of a treatment group;
Figure 2 is a graph showing HOMAR-IR analysis of a treatment group;
Figure 3 is a graph showing plasma triglyceride concentration of a treatment group;
Figure 4 is a graph showing blood pressure of a treatment group;
Figure 5 is a graph showing plasma CRP concentration of a treatment group;
Figure 6 is a graph showing plasma fibrinogen concentration of a treatment group;
Figure 7 is a graph showing weight gain per day of a treatment group;

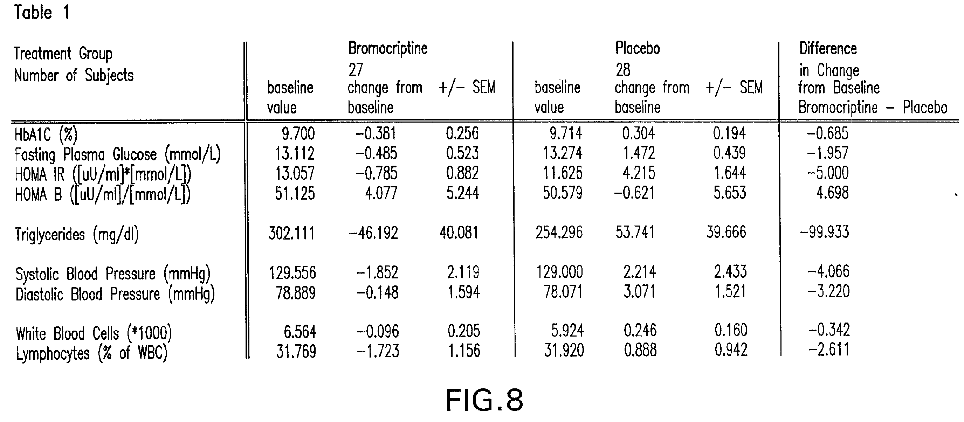

Figure 8 is a table showing the effects of bromocriptine treatment relative to placebo upon various metabolic parameters;

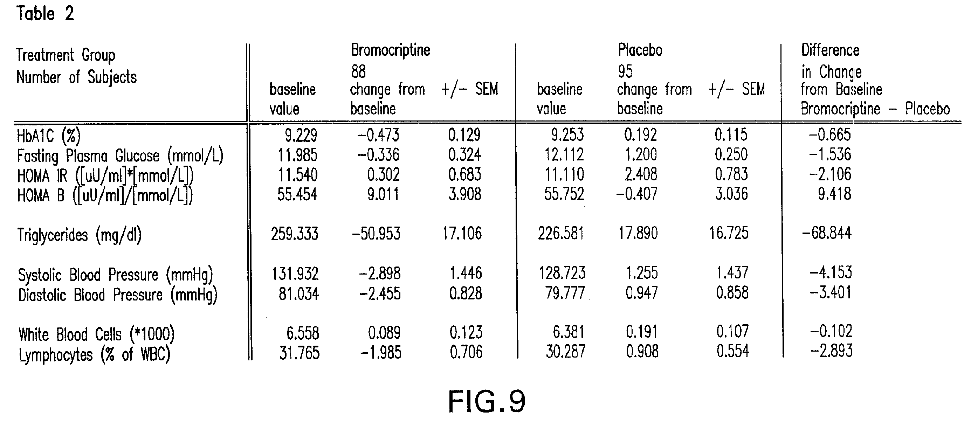

Figure 9 is another table showing the effects of bromocriptine treatment relative to placebo upon various metabolic parameters;

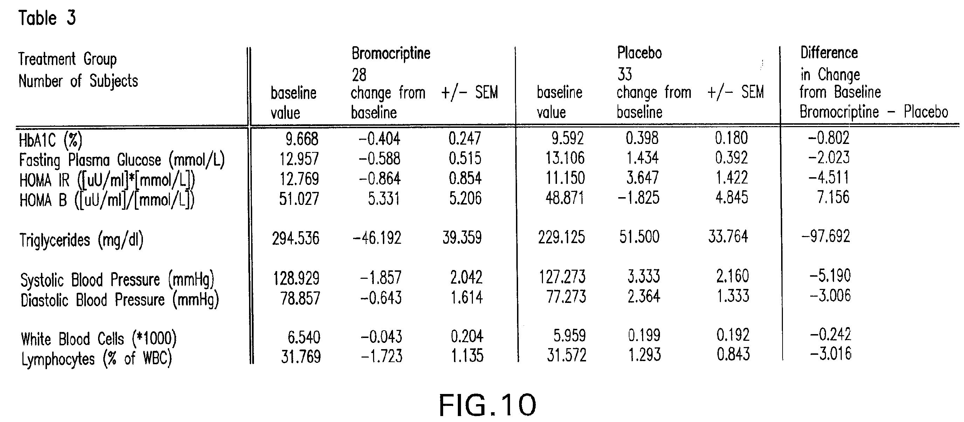

Figure 10 is another table showing the effects of bromocriptine treatment relative to placebo upon various metabolic parameters;

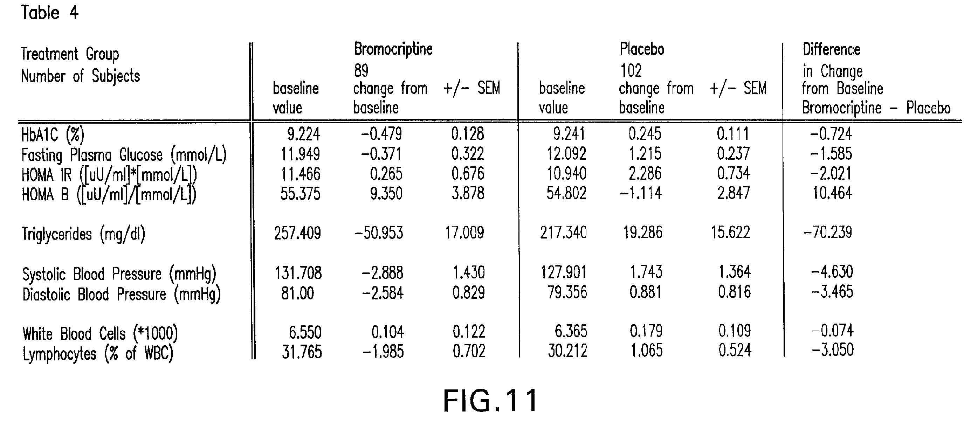

Figure 11 is another table showing the effects of bromocriptine treatment relative to placebo upon various metabolic parameters; and
Figure 12 is a graph showing blood pressure changes.

### DETAILED DESCRIPTION

In accordance with the present invention, a novel treatment for the Metabolic Syndrome (obesity, insulin resistance, hyperlipidemia, and hypertension), non-metabolic pathologies associated with MS (a pro-inflammatory state, a pro-coagulative state, a pro-oxidant state, and endothelial dysfunction), and for the treatment of arteriosclerosis and cardiovascular disease, all in subjects with or without Type 2 diabetes is presented. The treatment method of the invention also encompasses simultaneously treating one or more metabolic derangements of MS including hypertension, hypertriglyceridemia, insulin resistance and one or more non-metabolic derangements often associated with MS including a pro-inflammatory state, a pro-coagulative state, , pro-oxidant state, and endothelial dysfunction independently or associated with the Metabolic Syndrome. The treatment method of the invention also encompasses treating endothelial dysfunction associated with the Metabolic Syndrome or cardiovascular disease. The treatment methods comprise administering to a mammalian species in need of such treatment a pharmaceutical composition that simultaneously stimulates an increase in central dopaminergic neuronal activity level (particularly within neurons innervating the hypothalamus and the hypothalamus itself) and a decrease in central noradrenergic neuronal activity level (particularly within the brain stem region that innervates the hypothalamus and the hypothalamus itself). It has been unexpectedly discovered that increasing the ratio of dopaminergic neuronal to noradrenergic neuronal activity within the hypothalamus of the central nervous system improves the Metabolic Syndrome and/or Type 2 diabetes conditions, as well as the conditions of hypertension, hypertriglyceridemia, pro-inflammatory states, pro-coagulative states, pro-oxidant states, insulin resistance, and endothelial dysfunction associated with or independent from the Metabolic Syndrome. As defined herein, "neuronal activity" refers to either an increase or decrease in the synaptic neurochemical signal transmission of a neuron to another thereby affecting action potential. As defined herein, the term "pro-oxidant state" refers to an increase in the oxidizing capacity of components or molecular species within the blood or tissues.

In one embodiment, the method of the present invention includes administering to a subject in need of treatment for the Metabolic Syndrome or Type 2 diabetes a pharmaceutical composition comprising (1) at least one compound that stimulates an increase in central dopaminergic neuronal activity level in said subject, and (2) at least one compound that stimulates a decrease in central noradrenergic neuronal activity level in said subject. In an alternative embodiment, the pharmaceutical composition may include a single compound that stimulates an increase in central dopaminergic neuronal activity level as well as stimulates a decrease in central noradrenergic neuronal activity level. It is also contemplated that two, three, four, or more such compounds, each capable of simultaneously stimulating an increase in central dopaminergic neuronal activity level as well as stimulates a decrease in central noradrenergic neuronal activity level, may be used in the pharmaceutical composition. In all embodiments, however, the ratio of dopaminergic neuronal to noradrenergic neuronal activity within the hypothalamus is increased.

The increase in central dopaminergic neuronal activity level can take place by any mechanism. In preferred embodiments, the increase in central dopaminergic neuronal activity level occurs by including in the pharmaceutical composition at least one compound that stimulates an increase in central dopaminergic neuronal activity level. Preferably, such compounds include, but are not limited to, dopamine reuptake inhibitors, dopamine presynaptic transporter inhibitors, presynaptic dopamine release enhancers, post synaptic dopamine receptor agonists, dopamine synthesis stimulators, and/or dopamine catabolism inhibitors. Examples of useful compounds that stimulate an increase in central dopaminergic neuronal activity level include, but are not limited to, GBR-12935 (known as 1-[2-(diphenylmethoxy)ethyl]-4-(3-phenylpropyl)piperazine); BDNF (Brain Derived Neurotrophic Factor), quinpirole ((4aR-trans)-4,4a,5,6,7,8,8a,9-octahydro-5-propyl-1H-pyrazo10[3,4-g]quinoline); SKF38393 (1-phenyl-7,8-dihydroxy-2,3,4,5-tetrahydro-1H-3-benzazepine hydrochloride); deprenyl (also known as "Selegiline"); apomorphine, pramipexole (sold commercially under the name "Mirapex"), GBR-12909 ("Vanoxerine", 1-2-(bis(4-fluorophenyl)-methoxy)-ethyl-4-(3-phenylpropyl)piperazine); and combinations thereof.

The inhibition of noradrenergic neuronal activities may also be accomplished via any mechanism. In preferred embodiments, stimulation of a decrease in central noradrenergic activity level occurs by administration of at least one compound that results in a decrease in central noradrenergic activity level. Preferably, such compounds include, but are not limited to, postsynaptic noradrenergic receptor blockade compounds, inhibitors of noradrenalin release, inhibitors of noradrenalin synthesis, activators of noradrenalin presynaptic reuptake, and activators of noradrenalin catabolism presynaptically and in the synapse. Examples of useful compounds that decrease central noradrenergic activity level include, but are not limited to, prazosin (1-(4-amino-6,7-dimethoxy-2-quinazolinyl)-4-(2-furanylcarbonyl)piperizine): propranolol (1-(isopropylamino)-3-(1-naphthyloxy)-2-propanol); clonidine (2-(2,6-dichloroanilino)-2-imidazoline); fusaric acid (5-butyl-2-pyridinecarboxylic acid; 5-butylpicolinic acid); dopamine; phenoxybenzamine; phentolamine, (3-[[(4,5-dihydro-1H-imidazol-2-yl)methyl](4-methylphenyl)amino]phenol; 2-[N-(m-hydroxyphenyl-p-toluidineomethyl)imidazoline); guanfacine (sold under the brand name "Tenex"); and combinations thereof.

As indicated above, the method of the invention may also include administration of a pharmaceutical composition that includes a single or individual compound that simultaneously stimulates an increase in central dopaminergic neuronal activity level and a decrease in central noradrenergic neuronal activity level. Examples of such compounds include catecholamine modifiers, such as dopamine. Dopamine D2 receptor agonists may be classified in this category as they stimulate postsynaptic dopamine D2 receptors and inhibit release of norepinephrine presynaptically. However, dopamine D2 receptor agonists will possibly be susceptible to desensitization (reduced dopaminergic function) in part as a consequence of D2 agonist binding to presynaptic dopamine D2 receptor sites on dopaminergic neurons and thereby reducing dopamine release.

The compounds of the invention are preferably administered internally, e.g., orally, subcutaneously, transdermally, sublingually, transmucosally, or intravenously, in the form of conventional pharmaceutical compositions, for example in conventional enteral or parenteral pharmaceutically acceptable excipients containing organic and/or inorganic inert carriers, such as water, gelatin, lactose, starch, magnesium stearate, talc, plant oils, gums, alcohol, Vaseline, or the like. The pharmaceutical compositions can be in conventional solid forms, for example, tablets, dragees, suppositories, capsules, or the like, or conventional liquid forms, such as suspensions, emulsions, or the like. If desired, they can be sterilized and/or contain conventional pharmaceutical adjuvants, such as preservatives, stabilizing agents, wetting agents, emulsifying agents, buffers, or salts used for the adjustment of osmotic pressure. The pharmaceutical compositions may also contain other therapeutically active materials. The pharmaceutical compositions of the invention can be made using conventional methods know in the art of pharmaceutical manufacturing.

The pharmaceutical compositions of the invention should include an amount of the compound(s) of the invention effective for treatment of Metabolic Syndrome (obesity, insulin resistance, hyperlipidemia, and hypertension), non-metabolic pathologies associated with MS (a pro-inflammatory state, a pro-coagulative state, pro-oxidant state, and endothelial dysfunction), arteriosclerosis, and/or cardiovascular disease, all in subjects with or without Type 2 diabetes. The effective dosage will depend on the severity of the diseases and the activity of the particular compound(s) employed, and is thus within the ordinary skill of the art to determine for any particular host mammal or other host organism. Suitable dosages may be, for example, in the range of about 0.001 to about 100 mg per kg for a human being, and more preferably from about 0.1 to about 50 mg per kg for a human being.

The ratio of the compound(s) that stimulates an increase in central dopaminergic neuronal activity level to the compound(s) that stimulates a decrease in central noradrenergic neuronal activity level in the pharmaceutical composition generally ranges from about 500:1 to 1:500 on a weight-to-weight basis (w:w), and more preferably from about 100:1 to 1:100 on a weight-to-weight basis (w:w).

In further accordance with the method of the present invention, it has been surprisingly found that one or more of the metabolic disorders associated with Metabolic Syndrome may be treated by administering a central acting dopamine agonist (e.g., a dopamine D2 receptor agonist with or without a dopamine D1 receptor agonist), in particular hypertension, hypertriglyceridemia, a pro-inflammatory state, insulin resistance, and, optionally, obesity. Dopamine agonists have been used to treat diseases such as Parkinson's disease and diabetes. However, it has been surprisingly found that administering dopamine agonists to patients suffering from Metabolic Syndrome will alleviate their symptoms. An important advantage of the present invention is the ability to simultaneously treat multiple disorders of or associated with the Syndrome such as hypertension, insulin resistance, hypertriglyceridemia, a pro-inflammatory state, and optionally obesity.

As indicated above, in one embodiment, the present invention is directed to a method of treating insulin resistance, hypertension, a pro-inflammatory state, and hypertriglyceridemia. Fasting glucose of at least 110 mg/dl, plasma triglycerides at least 150 mg/dl, HDL cholesterol below 40 mg/dl in men or below 50 mg/dl in women, blood pressure at least 130/85 mm Hg, are also symptoms indicative of Metabolic Syndrome.

According to the method of the invention, treatment of one or more of the metabolic disorders associated with Metabolic Syndrome or of cardiovascular disease, cerebrovascular disease, or peripheral vascular disease includes administering to a patient suffering from Metabolic Syndrome or these vascular pathologies a therapeutically effective amount of a central acting dopamine agonist (e.g., a dopamine D2 receptor agonist with or without a dopamine D1 receptor agonist). Preferred central acting dopamine agonists include bromocriptine, quinpirole, quinerolane, talipexole, ropinirole, apomorphine, lisuride, terguride, fenoldopam, dihydroergotoxine, (hydergine), dihydroergocryptine, and combinations thereof. A most preferred central acting dopamine agonist is bromocriptine.

In accordance with the method of the invention, the central acting dopamine agonist is preferably administered internally, e.g., enteral or parenteral administration such as orally, transmucosally, sublingually, transdermally, or intravenously, in the form of conventional pharmaceutical compositions, for example in conventional enteral or parenteral pharmaceutically acceptable excipients containing organic and/or inorganic inert carriers, such as water, gelatin, lactose, starch, magnesium stearate, talc, plant oils, gums, alcohol, petroleum jelley, or the like. The pharmaceutical compositions can be in conventional solid forms, for example, tablets, dragees, suppositories, capsules, or the like, or conventional liquid forms, such as suspensions, emulsions, or the like. If desired, they can be sterilized and/or contain conventional pharmaceutical adjuvants, such as preservatives, stabilizing agents, wetting agents, emulsifying agents, buffers, or salts used for the adjustment of osmotic pressure. The pharmaceutical compositions may also contain other therapeutically active materials. The pharmaceutical compositions of the invention can be made using conventional methods know in the art of pharmaceutical manufacturing.

Further in accordance with the method of the present invention, the compounds or pharmaceutical compositions should include an amount of central acting dopamine agonist that is effective for treatment of the Metabolic Syndrome, or hypertension, hypertriglyceridemia, a pro-inflammatory state, a pro-coagulative state, a pro-oxidant state, insulin resistance, and/or endothelial dysfunction, either associated with the Metabolic Syndrome or independent of it as well as for manifestations of such metabolic abnormalities including arteriosclerosis, cardiovascular disease, peripheral vascular disease (including renal vascular disease), cerebrovascular disease, or congestive heart failure. The effective dosage of pharmaceutical composition and/or central acting dopamine agonist will depend on the severity of the diseases and the activity of the particular compound(s) employed, and is thus within the ordinary skill of the art to determine for any particular host mammal or other host organism. Suitable dosages of central acting dopamine agonist may be, for example, in the range of about 0.001 to about 0.2 mg per kg for a human being, and more preferably from about 0.01 to about 0.05 mg per kg for a human being. For oral tablets, the ratio of bromocriptine to carriers on a weight by weight basis is about 1 mg bromocriptine per 90 mg of tablet.

Respecting the treatment of cardiovascular, cerebrovascular, or peripheral vascular disease, individuals symptomatic of or diagnosed with such disorders may utilize such dopamine agonist therapy to inhibit the progression of or reverse the pathologic consequences of these existing vascular disorders. Consequently, use of central acting dopamine agonists for treatment of the metabolic disorders described herein represents a continuum of possible intervention times along the chronological development and worsening of such metabolic and vascular disorders from the time point of observable biomarkers of impending arteriosclerosis or vascular disease (a pro-inflammatory state, a pro-coagulative state, a pro-oxidant state, and/or endothelial dysfunction with or without hypertension, hypertriglyceridemia, and insulin resistance) to overt vascular disease.

Multiple circadian central neural oscillations govern the regulation and coordination of multiple physiological (e.g., metabolic) events in the periphery as a function of their circadian phase relationship, described in patent # 5,468,755 and herein incorporated in entirety by reference. One such circadian rhythm governing metabolic status is the central (hypothalamic) circadian rhythm of dopaminergic activity. It has previously been observed that phase shifts in the circadian rhythm of central dopaminergic activities influenced the status of obesity or diabetes. However, it has now been surprisingly found that phase shifts away from the healthy normal circadian rhythm of central or hypothalamic dopaminergic activity by environment, diet, stress, genetics and/ or other factors are somehow also operative in a much different and broader physiological regulatory system and potentiate and lead to the multiple complex metabolic pathologies of and associated with metabolic syndrome as described herein. Furthermore, it has now been found that resetting these aberrant central dopaminergic circadian rhythms back towards that of the healthy normal state results in simultaneous improvements in the multiple complex pathologies of and associated with metabolic syndrome as described herein. As described above, metabolic syndrome and its associated pathologies represent a different pathology from diabetes or obesity, the cause of which is unknown. However, subjects with metabolic syndrome have much greater risk of developing cardiovascular disease than subjects without the syndrome. Inasmuch as obesity and type 2 diabetes are not always associated with metabolic syndrome and vice versa, it is clear that this major health risk represents a separate and unique metabolic state with unique characteristics. Adjusting the circadian rhythm of central dopaminergic activities by various means may be employed to reduce the many pathologies of and associated with this syndrome, for example aberrant vascular tone, vascular health, endothelial function, glucose and lipid metabolism, immune system functions specifically influencing the vasculature, insulin action, and blood coaguability. This same circadian dopaminergic resetting methodology may also be utilized to treat cardiometabolic risk, a cluster of physiological pathologies of common or discordant origin that converge to increase risk of cardiovascular disease. These risk factors include those of metabolic syndrome, but also inflammation, endothelial dysfunction, hypercholesterolemia, diabetes, obesity, smoking, gender, and age. Rather than just increasing dopaminergic activity with central dopamine agonists to improve metabolic syndrome, cardiometabolic risk and their associated pathologies, one may better influence these conditions by timing the administration of such dopamine agonists to coincide with the daily peak in central dopamienrgic activities of healthy subjects of the same species to derive maximal benefit from such dopamine agonist therapy in treating these conditions.

In further accordance with this invention, the use of dopamine agonists to treat the Metabolic Syndrome (obesity, insulin resistance, hyperlipidemia, and hypertension), non-metabolic pathologies associated with MS (a pro-inflammatory state, a pro-coagulative state, pro-oxidant state, and/or endothelial dysfunction), arteriosclerosis, and/or cardiovascular disease, all in subjects with or without Type 2 diabetes, is applied during specific daily intervals to maximize the effectiveness of such treatment. Use of such centrally acting dopamine agonists for treatment of the metabolic and non-metabolic vascular disorders described herein may be potentiated by their administration at the appropriate time(s) of day. Circadian rhythms of dopaminergic activity within the central nervous system, and particularly the phase relations of these dopaminergic neuronal rhythms with other circadian neuronal activities such as serotonergic neuronal activities have been demonstrated to regulate peripheral glucose and lipid metabolism in a manner dependent upon the phase of the daily peak in circadian central dopaminergic activity. Consequently, increases in dopaminergic activity at particular times of day versus others produce maximal effectiveness in improving metabolic diseases and disorders such as type 2 diabetes, obesity, pre-diabetes, metabolic syndrome, cardiometabolic risk, hypertension, dyslipidemia, insulin resistance, hyperinsulinemia, hepatic steatosis, renal disease, cardiovascular disease, cerebrovascular disease, and peripheral vascular disease and biomarkers of impending vascular disease. As such, maximized successful treatment of these aforementioned pathologies and abnormalities may be accomplished by appropriately timed daily administration of centrally acting dopamine agonist(s). Because such dopamine agonist therapy attacks a root cause of these metabolic disorders (central dysregulation of global peripheral metabolism) it is possible to effectuate improvements in several metabolic pathologies in a simultaneous fashion that is not generally achievable by other conventional means that attack particular specific symptoms of metabolic disease for example hypertension or high cholesterol or hyperglycemia by acting at specific downstream peripheral targets such as biochemical pathways within liver or muscle. Such a treatment effect is currently lacking in the general armamentarium of therapeutics for metabolic diseases. Moreover, central dopamine agonist therapy may be coupled to direct or indirect peripheral acting therapeutic agents such as anti-diabetes agents, antihypertensive agents, cholesterol lowering agents, anti-inflammatory agents, or anti-obesity agents to produce additive improvements in metabolic disease such as obesity or type2 diabetes or particular aspects of metabolic disease such as hypertension associated with obesity or type 2 diabetes.

### EXAMPLES

The following examples are meant to illustrate, but in no way limit the present invention.

### EXAMPLE 1

Four different groups of animals exhibiting the Metabolic Syndrome and/or Type 2 diabetes are treated with either saline as control, central dopamine neuronal activity activator(s), central noradrenergic neuronal activity inhibitor(s), or a molecular entity or entities that is/are both a central dopaminergic neuronal activity activator and central noradrenergic neuronal activity inhibitor, respectively.

Relative to the control group the dopaminergic neuronal activator/noradrenergic neuronal activity inhibitor group exhibits the greatest improvement in metabolism (decrease in obesity, dyslipidemia, hypertension, insulin resistance, hyperinsulinemia, and/or hyperglycemia) that is also significantly better than that of either the dopaminergic activator or noradrenergic inhibitor groups. An unexpected synergism between the dopaminergic neuronal activity stimulator(s) and noradrenergic neuronal activity inhibitors(s) is observed relative to the effects on improvement of the Metabolic Syndrome and/ or Type 2 diabetes.

### EXAMPLE 2

Two groups of animals exhibiting the Metabolic Syndrome are treated with either a dopamine agonist such as bromocriptine or vehicle (control) for a period of time of approximately two weeks. The insulin sensitivity, plasma triglyceride level, blood pressure, pro-coagluant and pro-inflammatory factor level(s) of the animals are then determined. Relative to the control group, the dopamine agonist treated animals exhibit lower plasma triglyceride level, pro-coagulant and pro-inflammatory factor(s) level, blood pressure, and insulin resistance.

### EXAMPLE 3: Methods of Treating Vascular Related Disorders using Dopamine Receptor Agonists

**Background** Daily administration of bromocriptine mesylate in animal models of metabolic syndrome improves insulin resistance, glucose intolerance, dyslipidemia, elevated blood pressure, pro-inflammatory status and hypercoaguability. Clinical studies have likewise demonstrated that Cycloset therapy improves glucose intolerance, insulin resistance, glycemic control and dyslipidemia in obese subjects with insulin resistance or type 2 diabetes. However, the impact, if any, of Cycloset therapy upon cardiovascular adverse event rate in subjects with type 2 diabetes has not been previously studied in a large population. The current trial therefore investigated the influence of Cycloset on cardiovascular event rate and all-cause serious adverse events among subjects with type 2 diabetes currently treated with diet, oral hypoglycemic agents, and/or insulin.
**Methods** This trial was a 52 week, double blind, 2:1 randomized, multicenter study in type 2 diabetes patients receiving a diabetes therapeutic regimen consisting of diet or no more than two hypoglycemic agents or insulin with or without one additional oral agent that were randomized to treatment with Cycloset™ (titrated from 1.6 mg/day to a maximal tolerated dose up to 4.8 mg daily; n= 2,054), or placebo (n= 1,016) once daily in the morning shortly after awakening. The primary and secondary endpoints were time to first all-cause serious adverse event (SAE) and cardiovascular SAE (composite of myocardial infarction, stroke, coronary revascularization, or hospitalization for angina and congestive heart failure), respectively, which were adjudicated by an independent review committee. An analysis at week 24 of the between-treatment differences in HbAlc among a subpopulation of subjects receiving metformin and sulfonylurea and HbAlc of ≥ 7.5 and < 10.0 at baseline was also performed.
**Results** There were 176 Cycloset and 98 placebo subjects that experienced a SAE, yielding a rate ratio of 0.88 and a hazard ratio of all cause SAE of 1.023 (96% one sided confidence limit of 1.27). There were 31 (1.5%) cardiovascular SAEs in the Cycloset™ group and 31 (3.0%) such events in the placebo group resulting in a 42% reduction in cardiovascular outcomes in Cycloset treated subjects versus placebo (HR = 0.58, 95% CI: 0.35 - 0.96; P < 0.025). The incidence rate ratio for each of the components of the cardiovascular composite was less than 1.0. Among the pre-specified subpopulation of subjects, Cycloset (n= 121) treatment resulted in an HbAlc reduction of -0.674 from baseline versus an increase for placebo (n = 71) of 0.015 to give a placebo-adjusted change from baseline of -0.69 (P <0.0002). Of these Cycloset treated subjects, 39% (vs. 11% placebo) reached the ADA goal of HbAlc of ≤ 7.0 (P<0.0007) and 53% (vs. 21% placebo) experienced a minimum reduction in HbAlc from baseline of 0.7 (p<0.0001).
**Discussion** Cycloset significantly reduced the risk for the a priori adjudicated cardiovascular adverse event endpoint and was comparable to placebo for all other serious adverse events for the entire study population. Among the pre-specified subgroup of individuals inadequately controlled on metformin and sulfonylurea, 24 weeks of Cycloset therapy significantly improved glycemic control relative to placebo.. These results indicate that appropriately timed daily dopamine agonist therapy concurrently reduces risk of microvascular complications, via improving glycemic control and also reduces marcovascular events within one year of therapy. The ability to significantly reduce microvascular and macrovascular disease in subjects with type 2 diabetes is a favorable and rather unique therapeutic profile for a single pharmaceutical agent.

### EXAMPLE 4

Patients with type 2 diabetes have an increased risk of cardiovascular disease (CVD). Available evidence suggests that timed administration of Cycloset, a D2 receptor agonist, acts centrally to increase early morning dopaminergic activity in subjects with diabetes which in turn improves many cardiometabolic abnormalities such as hypertension, insulin resistance, hypertriglyceridemia, and inflammation. The Cycloset Safety Trial was a prospective, multicenter, double blind, placebo-controlled 52 week study of 3,070 subjects with type 2 diabetes. Subjects were randomized 2:1 to either Cycloset or placebo, respectively in addition to their other glucose-lowering and cardiovascular medications. Cycloset had a statistically significant benefit on the pre-specified CVD composite endpoint of myocardial infarction (MI), stroke, coronary revascularization, hospitalization for angina or congestive heart failure (42% risk reduction [RR]; p = 0.036). This analysis includes a post-hoc analysis from the Cycloset Safety Trial that assesses the effect of Cycloset on the time to first occurrence of major adverse cardiovascular events (MACE) defined as the composite of MI, stroke and CVD death and additional planned analysis of the influence of Cycloset on the CVD composite endpoint stratified by the baseline median HbA1c. CVD risk estimates were estimated as a hazard ratio [HR] and 95% confidence interval [CI] on the basis of the Cox proportional-hazards regression.
Cycloset had a statistically significant beneficial effect on the risk of myocardial infarction, stroke and CVD death (55% RR; p = 0.049). Among subjects with HbAlc ≤ 7.0 there were fewer CVD events on Cycloset (15, n = 1219) compared to placebo (18, n = 615). For those with HbAlc > 7.0 CVD events were also less on Cycloset (16, n = 830) compared to placebo (12, n = 400). The HR of the CVD composite endpoint for subjects with a baseline HbAlc of ≤ 7.0 or > 7.0 was 0.48 (95% CI 0.24 - 0.95) or 0.74 (95% CI 0.35 - 1.56), respectively. Additionally, the beneficial reduction in the CVD composite endpoint was apparent regardless of age, gender or race. Cycloset significantly reduced the risk for myocardial infarction, stroke, and cardiovascular death. The macrovascular risk reduction for the pre-specified cardiovascular composite endpoint was apparent even among subjects with good glycemic control.

The effects of timed bromocriptine treatment on CVD events described in the subject population within examples 3 and 4 above are exceedingly surprising and unexpected given the magnitude of the response and the short duration of exposure to timed bromocriptine to elicit this response. This response is among the largest if not the largest reduction in one year's time in cardiovascular events (composite or all CVD events or major events of myocardial infarction, stroke, and CVD death) ever reported in a large randomized clinical study testing for drug induced reduction in pre-specified endpoints of CVD for such a patient population. It must be noted that these subjects on average were in good metabolic control respecting blood pressure, blood glucose level, plasma triglyceride, total cholesterol and LDL cholesterol levels at the start of the trial as most were on medications for these metabolic parameters (statins, anti-diabetes medications, anti-hypertensive medications). Nonetheless, Cycloset (a quick release formulation of bromocriptine mesylate) given once in the morning was still able to reduce CVD events by 42% to 55% relative to placebo treated subjects. Other therapies to treat CVD such as statin therapy and anti-hypertensive therapy do no produce such marked results in a one year time span of exposure (for example, Colhoun H et al, Lancet 364; 685-696, 2004) in a similar patient population. Moreover, these Cycloset effects *cannot* be attributed to marked reductions in MS defined metabolic parameters such as blood pressure, triglycerides, glucose, or cholesterol (total, HDL, or LDL) as none of these parameters was influenced to a degree that would impact CVD. Blood pressure was decreased by 1-2 mm HG and plasma triglycerides, cholesterol (total, HDL, or LDL) and glucose were not clinically affected by Cycloset treatment, relative to placebo. These findings are in good agreement with the general tenet of this invention that timed dopamine agonist therapy influence on non-metabolic parameters such as a vascular pro-inflammatory state, pro-oxidant state, pro-coagulative state, and endothelial dysfunction with or without impact on plasma hypertriglyceridemia, high cholesterol, high glucose, or high blood pressure can effectuate large decreases in CVD events, likely by impact on arteriosclerosis. Again, these findings and conclusions are consistent with the observations that drugs that dramatically lower plasma lipids and blood pressure (35 -50% and 10-15 mmHG, respectively) do not produce such marked reductions in CVD events within just a one year time period of exposure. Taken as a composite, these Cycloset data along with data of lipid and blood pressure lowering drugs on CVD event rate, indicate that timed dopamine agonist therapy, while capable of simultaneously treating hypertriglyceridemia, hypertension and insulin resistance, is acting at other sites besides those defined by metabolic syndrome to beneficially impact CVD events.

### EXAMPLE 5

Several studies of older (16 weeks of age) male hypertensive insulin resistant SHR rats known to have arterial stiffness (arteriosclerosis) were conducted to determine the effect of timed bromocriptine on blood pressure, obesity, insulin resistance, hyperlipidemia, biomarkers of a pro-inflammatory state, a pro-oxidant state, a pro-coagulative state, endothelial dysfunction, and arterial stiffness (arteriosclerosis marker). Notably, these animals do not have signs of atherosclerosis so the effects if any on arteriosclerosis cannot be confused in any way with an effect on atherosclerosis. These animals were allowed to feed and drink ad libitum while held on 12 hour daily photoperiods and were randomized to different groups for treatment with bromocriptine (10 mg/kg) at either 1 hour after light onset (HALO), 7 HALO, 13 HALO, or 19 HALO or vehicle treatment for 14 days. Animals were tested for treatment effects on blood pressure, obesity, insulin resistance, hyperlipidemia, biomarkers of a vascular pro-inflammatory state, pro-oxidant state, pro-coagulative state, endothelial dysfunction, and arterial stiffness (arteriosclerosis marker) at 12 to 14 days after treatment initiation. It was found that treatment with bromocriptine at 13 HALO (onset of the locomotor activity rhythm in these nocturnal rodents) reduced high systolic and diastolic blood pressure (by approximately 15%), obesity (by 42%), insulin (by 55%), glucose (by 11%) insulin resistance (by approximately 50%), hyperlipidemia (by approximately 10%), a vascular pro-inflammatory state (decrease in adipose TNFalpha protein per cellular fat mass, by 40% and in adipose MCP-1 protein per cellular fat mass, by 42%, in plasma CRP level by approximately 10%, and an increase in plasma adiponectin level of 10-30%), a pro-oxidant state (decrease in elevated aorta eNOS protein level, 22% and aorta iNOS protein level, 17%) a procoagluative state (increase in clotting time and decrease in plasma endothelin-1 (30%) and fibrinogen levels), and endothelial dysfunction (decrease in elevated aorta eNOS protein level, 22% and a 16% increase in aorta soluable guanyl cyclase protein level). Consistent with such findings and of major import, such treatment also markedly reduced arterial stiffness (arteriosclerosis) in measured in the aortas of these animals after only 14 days of treatment. The magnitude and breadth of such effects could not be produced by bromocriptine treatment at any of the other test times of day. Consequently, dopamine agonist therapy at the onset of locomotor activity versus other times of day in an animal model of arteriosclerosis demonstrated the most effective beneficial impact on both non-metabolic derangements of arteriosclerosis as well as on arteriosclerosis itself. It is important that in order to effectuate the maximum beneficial response to dopamine agonist in the above metabolic and non-metabolic parameters that the dopamine agonist be largely removed from the circulation within an approximate 6-12 hour window from the time of its peak concentration in the blood after it is administration..

### EXAMPLE 6

Based on the findings of previous experiments demonstrating that dopamine agonists are most effective at treating high blood pressure (hypertension), obesity, insulin resistance, hyperlipidemia, and biomarkers of a pro-inflammatory state, a pro-oxidant state, a pro-coagulative state, endothelial dysfunction, and arterial stiffness (arteriosclerosis marker) when administered at the onset of the locomotor activity rhythm in rats (13 HALO), further studies were conducted with a variety of different dopamine receptor agonists administered at this same time of day (13 HALO) to different groups of hypertensive, insulin resistant SHR rats with arterial stiffness (arteriosclerosis), respectively. Different groups of such SHR rats were treated with either bromocriptine (10 mg/kg) with or without SKF38393 (1 mg/kg), pergolide (0.1 mg/kg), terguride (2 mg/kg), talipexole (0.3 mg/kg), quinelorane (0.15 mg/kg) or vehicle for 9 to 14 days. At the end of treatment assays of high blood pressure, obesity, insulin resistance, hyperlipidemia, and biomarkers of a pro-inflammatory state, a pro-oxidant state, a pro-coagulative state, endothelial dysfunction, and arterial stiffness (arteriosclerosis marker) were conducted. It was found that, although these different dopamine agonists displayed varying degrees (magnitude) of activity in treating high blood pressure, obesity, insulin resistance, hyperlipidemia, and biomarkers of a pro-inflammatory state, a pro-oxidant state, a pro-coagulative state, endothelial dysfunction, and arterial stiffness (arteriosclerosis marker) relative to each other, relative to vehicle controls each was generally effective in treating high blood pressure, obesity, insulin resistance, hyperlipidemia, and biomarkers of a pro-inflammatory state, a pro-oxidant state, a pro-coagulative state, endothelial dysfunction, and arterial stiffness (arteriosclerosis marker). The reductions induced by dopamine agonists upon arterial stiffness was rather marked. Terguride was found to be a particularly strong anti-hypertensive agent reducing systolic and diastolic blood pressure by 30%. As can be seen from the structures of these molecules depicted below, they are very dissimilar stemming from ergot alkaloid, ergoline, non-ergot related and benzazepine parent structures. The major commonality, possibly the only significant commomality among them, is that they are dopamine D2 and/or D1 receptor agonists. 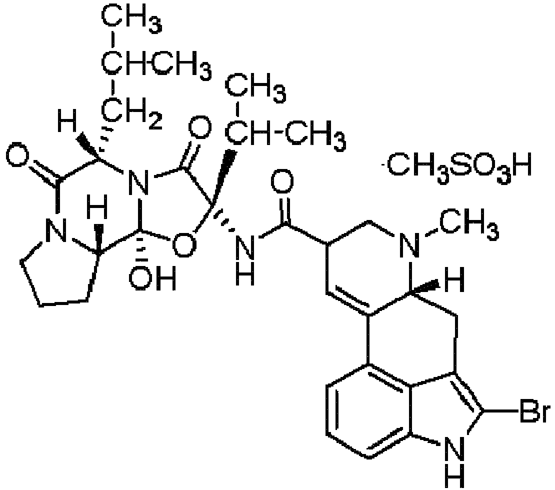
(+)-2-Bromo-12'-hydroxy-2'-(1-methylethyl)-5'-(2-methylpropyl)ergotaman-3',6'-18-trione methanesulfonate salt
(+)-Bromocriptine methanesulfonate salt
Bromocriptine mesylate salt
2-Bromo-α-ergocryptine methanesulfonate salt

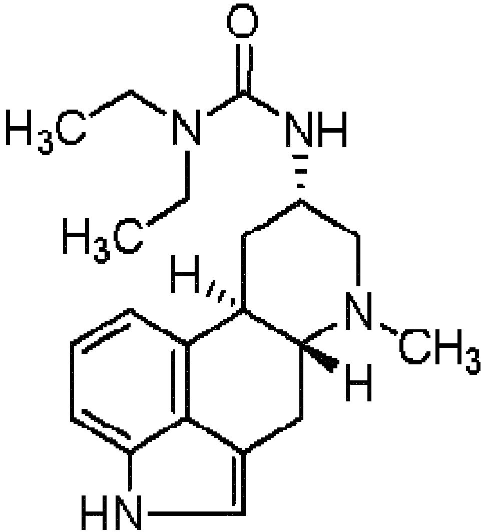

S(+)-N,N-Diethyl-N'([8α]-6-methylergolin-8-yl)urea
S(+)-Terguride (5R, 8S, 10R)
S(+)-Terguride

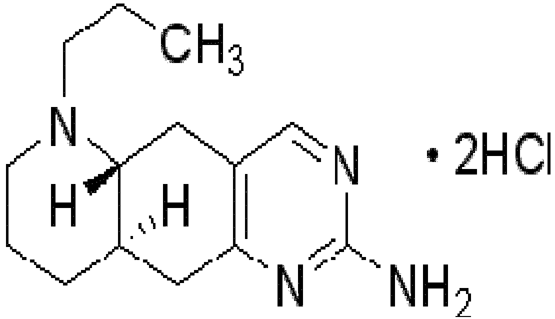

(5aR-trans)-5,5a,6,7,8,9,9a,10-Octahydro-6-propylpyrido[2,3-g]quinazolin-2-amine dihydrochloride Quinelorane dihydrochloride

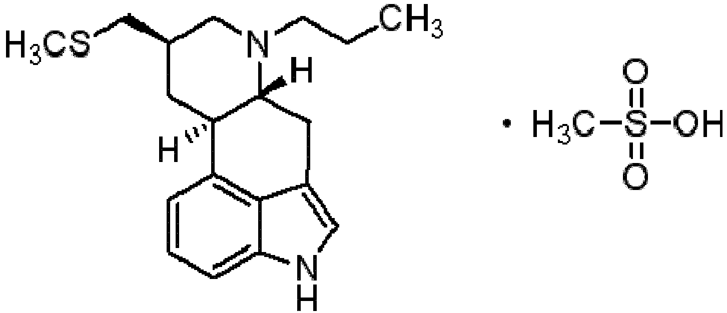

8β-[(Methylthio)methyl]-6-propylergoline methanesulfonate salt
Pergolide methanesulfonate salt
Pergolide mesylate salt

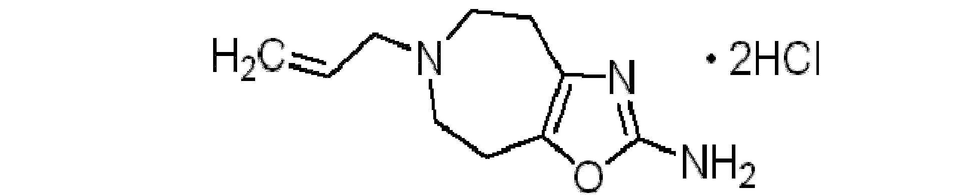

5,6,7,8-Tetrahydro-6-(2-propenyl)-4H-thiazolo[4,5-d]azepine-2-amine dihydrochloride Talipexole
B-HT 920 dihydrochloride

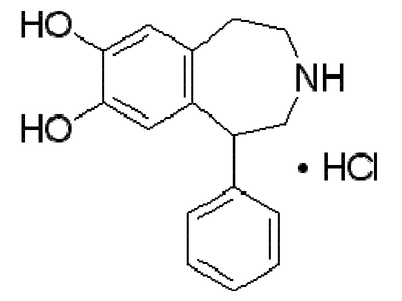

(±)-1-Phenyl-2,3,4,5-tetrahydro-(1H)-3-benzazepine-7,8-diol hydrochloride
(±)-SKF-38393 hydrochloride
D₁ Dopamine receptor agonist

Substituted 1-phenyl-3-benzazepines form a class of compounds possessing potent and selective affinity for the D1 DA receptor. 7,8-Dihydroxy-1-phenyl-2,3,4,5-tetrahydro-1H-3-benzazepine (SKF 38393) and its 6-halo analogues are potent and selective D1 receptor agonists.

### EXAMPLE 7: Effects of bromocriptine treatment upon clinical disorders and pathologies associated with metabolic syndrome in humans

This Example shows that treatment of metabolic syndrome subjects with bromocriptine (a dopamine D2 receptor agonist) at the onset of locomotor activity in the morning simultaneously reduces insulin resistance, hypertriglyceridemia, markers of a hyper-inflammatory state associated with increased cardiovascular risk, and blood pressure.

### Methods

Obese, type 2 diabetic subjects poorly controlled on sulfonylurea therapy and giving informed written consent to participate in a double blind, placebo controlled trial of the influence of bromocriptine to improve glycemic control were randomized to treatment with either bromocriptine or placebo and treated for 24 weeks. Subjects were eligible only if they had maintained a stable dose use of sulfonylurea and hyperlipidemic drugs for 60 and 30 days prior to randomization, respectively. The subjects in Tables 1 and 2 were exposed to either a) maximal doses of sulfonylurea; glipizide-15 mg/day, glyburide-10 mg/day, chlorpropamide- 350 mg/day, and tolbutamide- 500 mg/day or b) less than or equal to maximal doses of sulfonylurea, respectively. The subjects in this analysis fulfill criteria for metabolic syndrome (any three of the following: fasting glucose >/= 110 mg/dl, fasting triglyceride >/= 150 mg/dl, fasting HDL < 40 for males and < 50 for females, blood pressure >/= 130/ >/=85 and obese). At study initiation subjects took 0.8 mg tablet of bromocriptine or a placebo tablet in the morning after awaking for 1 week. Each subsequent week for an additional 5 weeks, the tablet number was increased by 1 per week until the maximum tolerated dose of between 1.6 and 4.8 mg per day was achieved while maintaining the dosing time at awakening in the morning. The subjects were then maintained on the maximum tolerated dose of bromocriptine or placebo for the remainder of the trial until completion at 24 weeks from randomization date. Prior to initial dosing and then again at the end of the study, blood samples were taken for analyses of fasting % glycated hemoglobin (HbA1c), insulin, glucose, triglyceride, and white blood cell (WBC) numbers and sub-fraction percentages; blood pressure measures were also recorded. A determination of insulin resistance and insulin secretory function was also made in these subjects at the beginning and end of the trial. Insulin resistance was determined by the HOMA-IR method [Fasting Glucose (mmol/l)*Fasting Insulin (uU/ml)/22.5] and insulin secretory function was determined by the HOMA-B method [20*Fasting Insulin(uU/ml)/Fasting Glucose (mmol) - 3.5].

### Results

Table 1 delineates the effects of bromocriptine treatment relative to placebo upon various metabolic parameters including blood HbA1c, fasting plasma glucose, triglyceride, WBC number and subfraction percentages, and blood pressure in metabolic syndrome subjects with type 2 diabetes. Determinations of treatment differences between bromocriptine and placebo-treated subjects for HOMA-IR and HOMA-B are also included in this table.

The results demonstrate that bromocriptine therapy for 24 weeks in metabolic syndrome - type 2 diabetic subjects simultaneously improves glycemic control (as evidenced by a reduction in HbAlc levels) and improves (reduces) hyperglycemia, insulin resistance (reduces HOMA-IR values), blood pressure, plasma triglycerides, and blood WBC and lymphocyte numbers, relative to placebo controls. The reductions in WBC and lymphocyte numbers remain within the clinically normal range.

### Discussion

Metabolic syndrome is a constellation of metabolic abnormalities that converge to increase cardiovascular risk. Among such subjects, insulin resistance, hyper-triglyceridemia, high blood pressure, and presence of pro-inflammatory immuno-status can interact to accelerate the development and progression of cardiovascular disease. While it is important to treat each of these pathologies, it would be beneficial from economic, clinical and practical perspectives to be able to treat this constellation of disorders with a single effective therapy. These study results indicate that a viable means of doing so is by the administration of a central acting dopamine agonist such as bromocriptine.

Bromocriptine treatment reduced the HOMA-IR value from 13.057 to 12.272 versus an increase from 11.626 to 15.841 in placebo controls, clearly indicating that insulin sensitivity had improved in response to treatment relative to placebo controls. Simultaneous with this effect, such treatment also improved the insulin secretory response (ability of the Beta cell to appropriately secrete insulin in response to circulating glucose levels) (HOMA-B increase from 51.125 to 55.202 for bromocriptine treated subjects versus a decrease from 50.579 to 49.928 for placebo controls). The bromocriptine-induced triglyceride improvement may also be expected to reduce other atherogenic factors in the blood of these subjects and reduce the risk of CVD. High blood pressure is a well known risk factor for CVD and bromocriptine therapy reduced both systolic and diastolic blood pressure in these subjects, relative to controls. Finally, sub-clinical increases in circulating WBC number and lymphocyte populations have been implicated as markers for pro-inflammatory stimulation of CVD. Bromocriptine treatment reduced the circulating numbers of WBC and lymphocytes while maintaining them within the normal range and this may be expected to be associated with reduced risk of CVD in metabolic syndrome subjects.

### EXAMPLE 8: Effects of bromocriptine treatment upon disorders and pathologies associated with metabolic syndrome in hypertensive, insulin resistant SHR rats

### Introduction

The male SHR rat is well defined as a rodent model of hypertension. When these animals are subjected to a "westernized diet" consisting of a high fat- high simple sugar composition, they develop severe insulin resistance and hypertriglyceridemia on top of their existing hypertension. Utilizing this diet-induced hypertensive and insulin resistant animal model system we describe a method for simultaneously improving (reducing) insulin resistance, hypertension, hypertriglyceridemia, pro-inflammatory factors potentiating cardiovascular risk, and a hyper-coagulative state utilizing a central acting dopamine agonist.

### Methods

Male SHR rats of 4-5 weeks age were housed 2 per cage and acclimated to our animal care facility and westernized diet for 4 weeks prior to the initiation of drug intervention studies. The animals were allowed free access to food ("westernized diet"; Research Diets Inc. # D12079B; 41% fat, 29% sucrose) and water ad libitum and were maintained at 72°F and on 14 hour daily photoperiods (light onset at 0600) for the duration of the acclimation and study periods. After the acclimation period (4 weeks), animals were randomized to treatment with vehicle or bromocriptine (5-10 mg/kg/day) at 13 hours after light onset (onset of daily locomotor activity rhythm) and treated for 28 days. On the seventeenth day of the study blood pressure measurements were made at approximately 12 hours after light onset. On the twenty-ninth day of the study animals were anesthetized (sodium pentobarbital 90/mg/kg) prior to cardiac puncture for blood sampling and then euthanized by additional sodium pentobarbital overdose (180/mg/kg). Plasma from blood samples were analyzed for glucose, insulin, triglyceride, C-Reactive Protein, and Fibrinogen levels.

### Results

Control animals exhibited metabolic syndrome conditions, as hypertension, hyperglycemia, and hypertriglyceridemia, as well as insulin resistance.

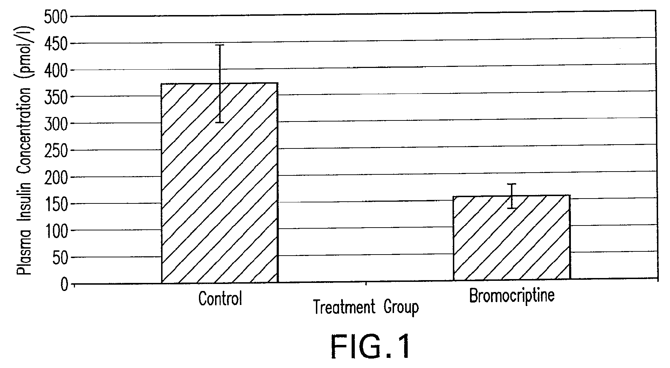

Figure 1 indicates that bromocriptine therapy reduces plasma insulin level by 59% relative to control animals.

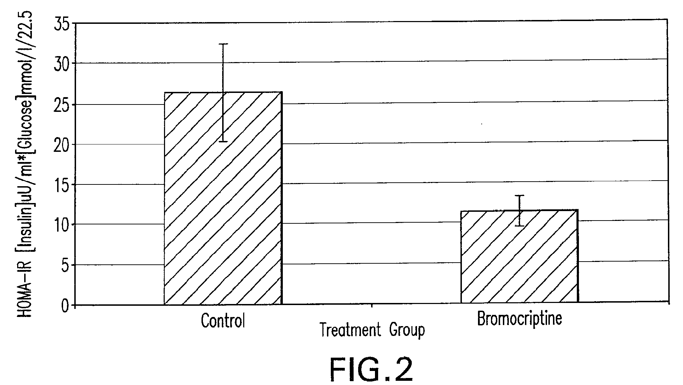

Figure 2 indicates that bromocriptine therapy reduces HOMA-IR by 55% relative to control animals.

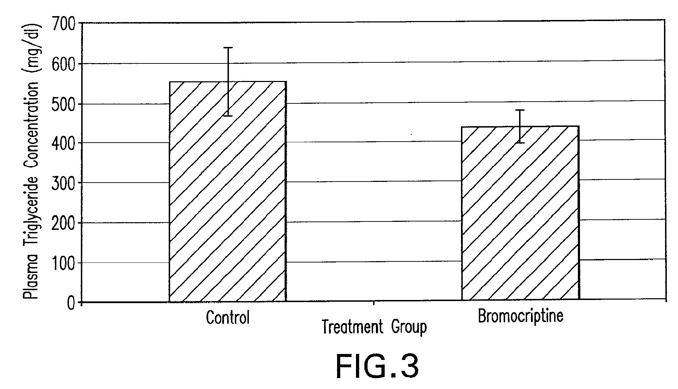

Figure 3 indicates that bromocriptine therapy reduces plasma triglyceride levels by 24% relative to control animals.

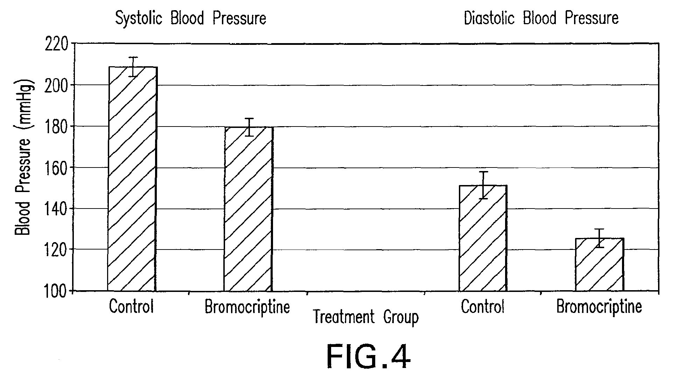

Figure 4 indicates that bromocriptine therapy reduces systolic blood pressure by 14% and diastolic blood pressure by 19% relative to control animals.

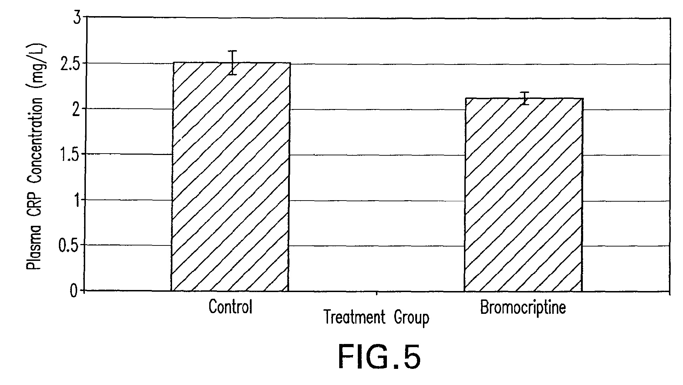

Figure 5 indicates that bromocriptine therapy reduces plasma C-Reactive Protein level by 16% relative to control animals.

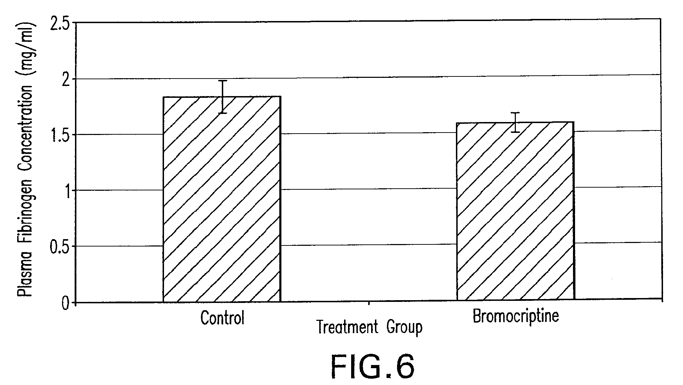

Figure 6 indicates that bromocriptine therapy reduces plasma fibrinogen level by 11% relative to control animals.

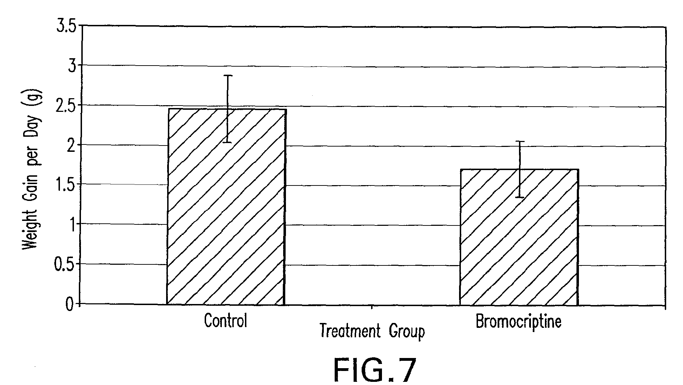

Figure 7 indicates that bromocriptine therapy reduces body weight gain by 29% relative to control animals.

### Discussion

Once daily bromocriptine therapy simultaneously improved hyperinsulinemia, insulin resistance (as evidenced by a decrease in the HOMA-IR value), hypertriglyceridemia, systolic and diastolic blood pressure, plasma C-Reactive Protein levels (index of inflammatory status), plasma fibrinogen levels (index of blood coagulation potential), and body weight gain. A reduction in insulin resistance, hypertriglyceridemia, a pro-inflammatory state, and a hyper-coagulative state in a simultaneous manner would potentiate a protective effect against the progression of cardiovascular disease. The bromocriptine-induced reduction in plasma fibrinogen level from the present study is consistent with another similar study of SHR rats wherein bromocriptine therapy for 2-4 weeks (5 mg/kg) increased clotting time (bleeding from a distal tail clip) relative to control, vehicle-treated animals. Taken in total, these findings demonstrate that a central acting dopamine agonist such as bromocriptine can function to simultaneously reduce weight gain, insulin resistance, hypertriglyceridemia, high blood pressure, a pro-inflammatory state, and a hyper-coagulative state among metabolic syndrome type animals. Moreover, these effects are not dependent upon calorie restriction or a particular nutritional diet. In fact, these results are observed in the face of a diet known to exacerbate these pathologies ("westernized diet"). That is to say, such treatment inhibits the effect of the westernized diet to fully induce these pathologies.

### EXAMPLE 9: Influence of Time-of-Day on the Blood Pressure Effect of Bromocriptine

### Methods

Male SHR rats of 8 weeks age were housed 2 per cage and acclimated to our animal care facility for 4 weeks prior to the initiation of drug intervention studies. The animals were allowed free access to food (rodent chow diet; Harlan) and water ad libitum and were maintained at 72°F and on 14 hour daily photoperiods (light onset at 0600) for the duration of the acclimation and study periods. After the acclimation period (4 weeks), different groups of animals were randomized to treatment with vehicle or bromocriptine (1 or 5 mg/kg/day) at 1 or 13 hours after light onset and treated for 7 days. On the eighth day of the study blood pressure measurements were made at approximately 5 hours after light onset.

### Results

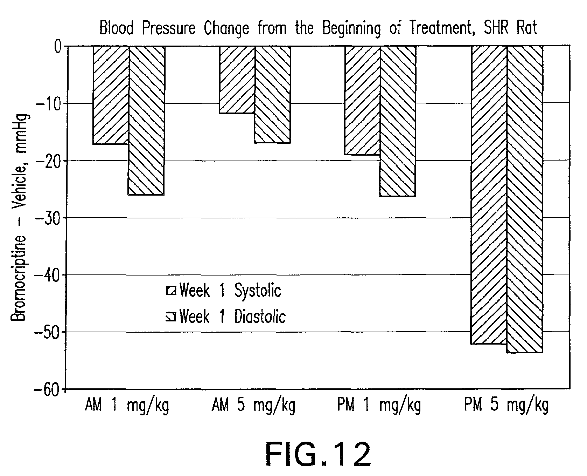

Relative to their respective controls, animals treated with 5 mg/kg/day bromocriptine at 13 HALO versus 1 HALO had greater reductions in blood pressure (Figure 12). More importantly, the reduction in blood pressure at 13 HALO persisted for 16 hours after treatment which is uncommon for such responses to bromocriptine, suggesting that the effect of the 13 HALO treatment was in response to the chronic treatment and long-lasting, suggesting that the effect of the 13 HALO treatment was in response to the chronic treatment and long-lasting. The blood pressure reduction at 1 HALO was only 4 hours after the bromocriptine administration.

### Discussion

These findings suggest that a daily variation in the responsiveness to central acting dopamine agonists such as bromocriptine in treating hypertension and therefore hypertensive - Metabolic Syndrome subjects may be critical and as of yet unappreciated in clinical practice.

### EXAMPLE 10: Effects of bromocriptine treatment upon metabolic disorders in subjects with Type 2 Diabetes

### Methods

Obese, type 2 diabetic subjects poorly controlled on sulfonylurea therapy and giving informed written consent to participate in a double blind, placebo controlled trial of the influence of bromocriptine to improve glycemic control were randomized to treatment with either bromocriptine or placebo and treated for 24 weeks. Subjects were eligible only if they had maintained a stable dose use of sulfonylurea and hyperlipidemic drugs for 60 and 30 days prior to randomization, respectively. The subjects in Tables 3 and 4 were exposed to either a) maximal doses of sulfonylurea; glipizide-15 mg/day, glyburide-10 mg/day, chlorpropamide- 350 mg/day, and tolbutamide- 500 mg/day or b) less than or equal to maximal doses of sulfonylurea, respectively. At study initiation subjects took 0.8 mg tablet of bromocriptine or a placebo tablet in the morning after awaking for 1 week. Each subsequent week for an additional 5 weeks, the tablet number was increased by 1 per week until the maximum tolerated dose of between 1.6 and 4.8 mg per day was achieved while maintaining the dosing time at awakening in the morning. The subjects were then maintained on the maximum tolerated dose of bromocriptine or placebo for the remainder of the trial until completion at 24 weeks from randomization date. Prior to initial dosing and then again at the end of the study, blood samples were taken for analyses of fasting % glycated hemoglobin (HbA1c), insulin, glucose, triglyceride, and white blood cell (WBC) numbers and sub-fraction percentages; blood pressure measures were also recorded. A determination of insulin resistance and insulin secretory function was also made in these subjects at the beginning and end of the trial. Insulin resistance was determined by the HOMA-IR method [Fasting Glucose (mmol/l)*Fasting Insulin (uU/ml)/22.5] and insulin secretory function was determined by the HOMA-B method [20*Fasting Insulin(uU/ml)/Fasting Glucose (mmol) - 3.5].

### Results

Tables 3 and 4 delineate the effects of bromocriptine treatment relative to placebo upon various metabolic parameters including blood HbA1c, fasting plasma glucose, triglyceride, WBC number and subfraction percentages, and blood pressure in metabolic syndrome subjects with type 2 diabetes. Determinations of treatment differences between bromocriptine and placebo-treated subjects for HOMA-IR and HOMA-B are also included in this table.

The results demonstrate that bromocriptine therapy for 24 weeks in type 2 diabetic subjects simultaneously improves glycemic control (as evidenced by a reduction in HbAlc levels) and improves (reduces) hyperglycemia, insulin resistance (reduces HOMA-IR values), blood pressure, plasma triglycerides, and blood WBC and lymphocyte numbers, relative to placebo controls. The reductions in WBC and lymphocyte numbers remain within the clinically normal range.

### Discussion

These results indicate that dopamine agonist therapy with bromocriptine can simultaneously improve type 2 diabetes, hypertension, hypertriglyceridemia, insulin resistance, and biomarkers of a pro-inflammatory state.

### Example 11

The hypertensive-obese state is a potent risk for cardiovascular disease. This condition is strongly coupled to insulin resistance and a pro-inflammatory humoral/vascular milieu. Previous studies have implicated an important role for circadian phase-dependent increase in hypothalamic dopaminergic tone in the maintenance of the lean, insulin sensitive condition. This study therefore investigated the effects of timed daily administration of bromocriptine, a dopamine D2 receptor agonist, on hypertension and humoral markers of a pro-inflammatory state in addition to effects on body fat store level and insulin resistance in SHRs. Sixteen week old SHRs maintained on 12 hour daily photoperiods were treated daily for 16 days with bromocriptine (10-15 mg/kg, i.p.) or vehicle at 1 hour before light offset. Measurements of blood pressure were taken 14 days after such treatment at 4 hours after light onset (16 hours after last bromocriptine injection) and animals were sacrificed on day 16 of the study for the analyses of body fat store level and humoral metabolic and immune factors. Bromocriptine treatment reduced systolic and diastolic blood pressures from 211 to 172 (P=0.025) and 159 to 119 (P=0.059), retroperitoneal body fat level by 33% (from 4.54 to 3.03 grams, P=0.004), plasma insulin level 45% from 289 to 160 pmol/L (P=0.0003), plasma glucose from 150 to 111 mg/dl (P=0.05), and HOMA-IR from 15.8 to 6.5 µU/ml*mmol/L (P=0.0015) relative to vehicle treated controls. Plasma C-reactive protein was reduced from 7 to 6.1 mg/L (P=0.0083). Plasma leptin level was also reduced by 58% (from 971 to 412, P<0.0001, pg/ml) by bromocriptine treatment. Moreover, in separate similarly designed studies of SHRs on chow or high fat-high sucrose diet, bromocriptine treatment also increased plasma level of adiponectin by 31 and 42 % (from 10.0 to 13.1, P=0.035 and 12.2 to 17.3, P=0.034, ng/ml), respectively. These findings indicate that in Spontaneously Hypertensive Rats timed daily administration of bromocriptine beneficially impacts several different metabolic and non-metabolic-related pathophysiologic activities predisposing to arteriosclerosis and cardiovascular disease. These findings further indicate that timed dopamine agonist therapy can simultaneously beneficially impact metabolic derangements and non-metabolic derangements of and associated with, respectively, metabolic syndrome. The above examples demonstrate that timed daily dopamine agonist therapy has the ability to simutaneoulsy treat a) several metabolic derangements including hypertension, hypertriglyceridemia, and insulin resistance and b) several non-metabolic derangements including a vascular pro-inflammatory state, a pro-coagulative state, a pro-oxidant state, and endothelial dysfunction, c) metabolic syndrome, c) type 2 diabetes, and also beneficially impact arteriosclerosis and cardiovascular disease progression. The substantial breadth and magnitude of these aspects of timed daily dopamine agonist therapy have heretofore been unrecognized. Aspects of available evidence prior to this disclosure actually argued against these findings respecting impact on vascular disease.

## Claims

1. A central acting dopamine agonist, for use in the treatment of at least one vascular disease in a patient suffering from or exhibiting biomarkers of said at least one vascular disease;
wherein said vascular disease is selected from the group consisting of arteriosclerosis, myocardial infarction, stroke, angina, and congestive heart failure;
wherein the central acting dopamine agonist is from dopamine D2 receptor agonists and/or dopamine D1 receptor agonists;
wherein the central acting dopamine agonist is bromocriptine;
wherein the bromocriptine is administered so as to effectuate peak plasma levels of the dopamine agonist between 0400 and 1200 hours of the day; and
wherein the bromocriptine bioavailability in the blood is reduced to within about 50% of the plasma peak value from about 2 to 6 hours after the end of the daily peak or plateau plasma level of bromocriptine.

2. The central acting dopamine agonist of claim 1, wherein said patient also suffers from one or more of Metabolic Syndrome, endothelial dysfunction, or Type 2 diabetes, and said method also treats Metabolic Syndrome and/or Type 2 diabetes.

3. The central acting dopamine agonist of claim 1, further comprising at least one of other anti-diabetes agent, anti-obesity agent, antihypertensive agent, anti-inflammatory agent, or cholesterol lowering agent.

4. The central acting dopamine agonist of claim 1, wherein said central acting dopamine agonist is further for use in treating obesity.

5. The central acting dopamine agonist of claim 1, wherein the amount of bromocriptine ranges from 0.001 mg per kg body weight to 2.0 mg per kg body weight.
